# EUROPÄISCHE PATENTSCHRIFT

(11) **EP 0 783 487 B1**
(45) Veröffentlichungstag und Bekanntmachung des Hinweises auf die Patenterteilung: **09.02.2000**
(21) Anmeldenummer: 95932774.3
(22) Anmeldetag: 19.09.1995
(51) Int. Cl.: C07C 317/24, C07C 49/84, C07C 245/08, C07D 319/06, C07D 339/08, A01N 41/10, A01N 35/02, A01N 43/28, A01N 43/32

(54) **2-AROYLCYCLOHEXANDIONE, VERFAHREN ZU IHRER HERSTELLUNG SOWIE IHRE VERWENDUNG ALS HERBIZIDE ODER DAS PFLANZENWACHSTUM REGULIERENDE MITTEL**
2-AROYLCYCLOHEXANDIONES, PROCESS FOR PREPARING THE SAME AND THEIR USE AS HERBICIDES OR PLANT GROWTH REGULATORS
2-AROYLCYCLOHEXANDIONES, LEUR PROCEDE DE PREPARATION ET LEUR UTILISATION COMME HERBICIDES OU COMME REGULATEURS DE LA CROISSANCE VEGETALE

(30) Priorität: 30.09.1994 DE 4434987
(43) Veröffentlichungstag der Anmeldung: 16.07.1997
(73) Patentinhaber: BASF AKTIENGESELLSCHAFT, 67056 Ludwigshafen (DE)
(72) Erfinder: KAST, Jürgen, D-67459 Böhl-Iggelheim (DE); VOSSEN, Marcus, D-68199 Mannheim (DE); VON DEYN, Wolfgang, D-67434 Neustadt (DE); ENGEL, Stefan, D-55286 Wörrstadt (DE); HILL, Regina, Luise, D-67346 Speyer (DE); KARDORFF, Uwe, D-68159 Mannheim (DE); OTTEN, Martina, D-67069 Ludwigshafen (DE); PLATH, Peter, D-67227 Frankenthal (DE); WALTER, Helmut, D-67283 Obrigheim (DE); WESTPHALEN, Karl-Otto, D-67346 Speyer (DE); MISSLITZ, Ulf, D-67433 Neutstadt (DE)
(86) Internationale Anmeldenummer: EP9503685
(87) Internationale Veröffentlichungsnummer: WO9610561

(56) Entgegenhaltungen:
- EP-A- 0 233 568
- EP-A- 0 319 075
- WO-A-91/01289

## Beschreibung

Die vorliegende Erfindung betrifft neue 2-Aroylcyclohexandione der Formel I 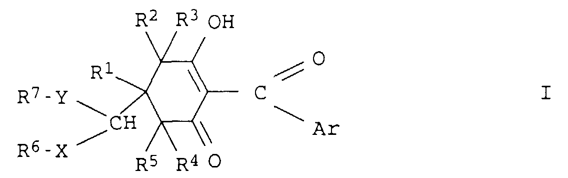 in der die Variablen folgende Bedeutung haben:
X und Y Sauerstoff;
Ar
   Phenyl oder einen 5- oder 6-gliedrigen Heteroarylring, wobei der Phenyl- oder Heteroarylring mindestens einen, höchstens jedoch vier Substituenten trägt, jeweils ausgewählt aus der Gruppe bestehend aus Halogen, Cyano, Nitro, -N=N-Ph, (C₁-C₄-Alkoxy)carbonyl, -N(R⁹)-COR¹⁰, -N(R⁹)-SO₂-R¹¹, -SO₂-N(R⁹)R¹⁰, -S(O)ₘ-R⁸, C₁-C₄-Alkyl, C₁-C₄-Alkoxy, C₁-C₄-Halogenalkyl und C₁-C₄-Halogenalkoxy, wobei die vier letztgenannten Reste ihrerseits ein oder zwei der folgenden Substituenten tragen können: C₁-C₄-Alkoxy, C₁-C₄-Alkylthio und/oder Cyano,
und wobei zwei benachbarte C-Atome des Phenyl- oder Heteroarylrings auch über eine Kette -C(R¹²)=C (R¹³)-C(R¹⁴)=C(R¹⁵)-, -Z¹-C(R¹²)=N-, -Z¹-N=C(R¹²)-, -Z¹-C(R¹²)=C(R¹³)-, -Z¹-C(R¹²)=C(R¹³)-C(R¹⁴, R¹⁵)-, -Z¹-C(R¹², R¹³)-C(R¹⁴, R¹⁵)-, -Z¹-C(R¹², R¹³)-C(R¹⁴, R¹⁵)-Z²-, -C(R¹², R¹³)-Z¹-C(R¹⁴, R¹⁵)-C(R¹⁶, R¹⁷)-, -Z¹-N(R²⁰)-Z²-, -Z¹-Z²-N(R²⁰)-, -Z¹-C(R¹², R¹³)-Z²-N(R²⁰)-, -Z¹-N(R²⁰)-Z²-N(R²¹)-, -N(R²¹)-Z¹-Z²-, -N(R²⁰)-Z¹-N=C(R¹²)-, -Z¹-C(R¹², R¹³)-C(=NOR²²)-, -Z¹-C(R¹², R¹³)-C(R¹⁴, R¹⁵)-C(=NOR²²)- oder Z¹-Z²-Z¹ verbrückt sein können, wobei
Z¹ und Z² unabhängig voneinander
   Sauerstoff, Schwefel, -SO-, -SO₂-, -CO-, -C(R¹⁸, R¹⁹)- oder -N(R²⁰)- bedeuten und wobei
R¹² bis R¹⁹ unabhängig voneinander für
   Wasserstoff, Halogen, Hydroxyl, C₁-C₄-Alkyl, C₁-C₄-Alkoxy, C₁-C₄-Alkylthio, C₁-C₄-Alkylamino, Di-(C₁-C₄-alkyl)amino oder Phenyl,
R²⁰ und R²¹ unabhängig voneinander für Wasserstoff, C₁-C₄-Alkyl, C₁-C₄-Alkoxy, (C₁-C4-Alkyl)carbonyl, Phenyl oder Benzoyl und
R²² für
   Wasserstoff, C₁-C₄-Alkyl, Allyl oder Phenyl stehen;
Ph steht für
   Phenyl, das unsubstituiert sein oder ein bis drei Substituenten tragen kann, ausgewählt aus der Gruppe bestehend aus Halogen, Cyano, Nitro, -S(O)ₙ-R²³, (C₁-C₄-Alkoxy)carbonyl, -SO₂-N(R²⁴)R²⁵, -N(R²⁴)-COR²⁵, -N(R²⁴)-SO2R²⁶, C₁-C₄-Alkyl, C₁-C₄-Alkoxy, C₁-C₄-Halogenalkyl und C₁-C₄-Halogenalkoxy, wobei die vier letztgenannten Reste ihrerseits ein oder zwei der folgenden Substituenten tragen können: C₁-C₄-Alkoxy, C₁-C₄-Alkylthio und/oder Cyano;
m und n stehen unabhängig voneinander für
   0, 1 oder 2;
R⁸ und R²³ stehen unabhängig voneinander für
   C₁-C₄-Alkyl oder C₁-C₄-Halogenalkyl, die beide ein oder zwei C₁-C₄-Alkoxy, C₁-C₄-Alkylthio- und/oder Cyanoreste tragen können;
R⁹, R¹⁰, R²⁴ und R²⁵ stehen unabhängig voneinander für
   Wasserstoff, C₁-C₄-Alkyl, C₁-C₄-Halogenalkyl oder Phenyl, das unsubstituiert sein oder ein bis drei Halogen-, C₁-C₄-Alkyl- und/ oder C₁-C₄-Alkoxyreste tragen kann;
R¹¹ und R²⁶ stehen unabhängig voneinander für
   C₁-C₄-Alkyl oder C₁-C₄-Halogenalkyl, die beide ein oder zwei Cyano-, Phenyl- und/oder Benzylreste tragen können;
R¹, R², R³ und R⁴
   Wasserstoff;
R⁵
   Wasserstoff;
R⁶ und R⁷
   unabhängig voneinander C₁-C₄-Alkyl, Benzyl oder zusammen eine Ethylen- oder Propylenkette, wobei jede Methyleneinheit ein oder zwei C₁-C₄-Alkylreste tragen kann;
sowie die landwirtschaftlich brauchbaren Salze von I und die Ester von I mit C₁-C₁₀-Carbon-, Sulfon-, Phosphonsäuren oder anorganischen Säuren.

Außerdem betrifft die Erfindung Verfahren zur Herstellung dieser Verbindungen, ihre Verwendung als Herbizide und zur Regulierung des Pflanzenwachstums sowie herbizide Mittel und Mittel zur Regulierung des Pflanzenwachstums, welche diese Verbindungen als wirksame Substanzen enthalten.

Des weiteren betrifft die Erfindung Verfahren zur Herstellung der herbiziden Mittel und Mittel zum Regulieren des Pflanzenwachstums, sowie Verfahren zur Bekämpfung von unerwünschtem Pflanzenwuchs und zur Regulierung des Pflanzenwachstums mit den Verbindungen I.

Herbizid wirksame 2-Aroylcyclohexandione mit einem Etherrest in 5-Position sind bereits aus EP-A 243 313, EP-A 319 075 und JP-A 03/052862 bekannt.

2-Aroylcyclohexandione mit Acetal- oder Thioacetalrest in 5-Position werden u.a. in der WO 91/01289 als Herbizide beschrieben.

Gemäß der EP-A 233 568 haben 5-(Dialkoxymethyl)-cyclohexan-1,3-dione mit einem Alkylcarbonyl-, Alkoxyalkylcarbonyl- oder Cyclopropylcarbonyl-Rest in 2-Position wachstumsregulierende Wirkung auf Pflanzen.

Die herbiziden und das Wachstum der Pflanzen regulierenden Eigenschaften der bekannten Verbindungen können jedoch, besonders bei kleinen Aufwandmengen und -konzentrationen, nur bedingt befriedigen.

Aufgabe der vorliegenden Erfindung war es deshalb, weitere 2-Aroylcyclohexandione mit verbesserten Eigenschaften bereitzustellen.

Demgemäß wurden die eingangs definierten 2-Aroylcyclohexandione der Formel I gefunden. Weiterhin wurden Verfahren zur Herstellung dieser Verbindungen, ihre Verwendung als Herbizide oder zur Regulierung des Pflanzenwachstums, Herbizide und das Wachstum der Pflanzen regulierende Mittel, die die Verbindungen I enthalten, Verfahren zur Herstellung dieser Mittel sowie Verfahren zur Bekämpfung von unerwünschtem Pflanzenwuchs und zur Regulierung des Pflanzenwuchses mit diesen Mitteln gefunden.

Die für die Substituenten R⁶ bis R²⁶ oder als Reste an Phenylringen oder Heterocyclen genannten organischen Molekülteile stellen - wie die Bedeutung Halogen - Sammelbegriffe für individuelle Aufzählungen der einzelnen Gruppenmitglieder dar. Sämtliche Alkyl- und Halogenalkyl-Teile können geradkettig oder verzweigt sein. Halogenierte Substituenten tragen vorzugsweise ein bis fünf gleiche oder verschiedene Halogenatome.

Im einzelnen bedeuten beispielsweise:
- Halogen: Fluor, Chlor, Brom oder Jod, vorzugsweise Fluor oder Chlor;
- C₁-C₄-Alkyl: Methyl, Ethyl, n-Propyl, 1-Methylethyl, n-Butyl, 1-Methylpropyl, 2-Methylpropyl oder 1,1-Dimethylethyl, vorzugsweise Methyl oder Ethyl;
- C₁-C₄-Halogenalkyl: C₁-C₄-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chorfluormethyl, Dichlorfluormethyl, Chlordifluormethyl, 2-Chlorethyl, 2-Fluorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, Pentafluorethyl, 3-Fluorpropyl, 2-Fluorpropyl, 2,2-Difluorpropyl, 2,3-Difluorpropyl, 3-Chlorpropyl, 2-Chlorpropyl, 2,3-Dichlorpropyl, 3-Brompropyl, 2-Brompropyl, 3,3,3-Trichlorpropyl, 3,3,3-Trifluorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl, vorzugsweise Trifluormethyl, Difluormethyl, 2,2,2-Trifluorethyl oder Pentafluorethyl;
- C₁-C₄-Alkoxy: Methoxy, Ethoxy, n-Propoxy, 1-Methylethoxy, n-Butoxy, 1-Methyl-propoxy, 2-Methylpropoxy oder 1,1-Dimethylethoxy, vorzugsweise Methoxy oder Ethoxy;
- C₁-C₄-Halogenalkoxy: C₁-C₄-Alkoxy wie vorstehend genannt, das partiell oder vollständig durch Fluor, Chlor und/oder Brom substituiert ist, also z.B. Difluormethoxy, Trifluormethoxy, Chlordifluormethoxy, Bromdifluormethoxy, 2-Fluorethoxy, 2,2-Difluorethoxy, 2,2,2-Trifluorethoxy, 2-Chlorethoxy, 2-Bromethoxy, 2-Iodethoxy, 2,2,2-Trichlorethyl, 2-Chlor-2-fluorethoxy, 2-Chlor-2,2-difluorethoxy, 2,2-Dichlor-2-fluorethoxy, 2,2,2-Trichlorethoxy, Pentafluorethoxy, 3-Fluorpropoxy, 2-Fluorpropoxy, 2,2-Difluorpropoxy, 2,3-Difluorpropoxy, 3-Chlorpropoxy, 2-Chlorpropoxy, 2,3-Dichlorpropoxy, 3-Brompropoxy, 2-Brompropoxy, 3, 3, 3-Trichlorpropoxy, 3,3,3-Trifluorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy, vorzugsweise Difluormethoxy oder Trifluormethoxy;
- C₁-C₄-Alkylthio: Methylthio, Ethylthio, n-Propylthio, 1-Methylethylthio, n-Butylthio, 1-Methylpropylthio, 2-Methylpropylthio oder 1,1-Dimethylethylthio, vorzugsweise Methylthio und Ethylthio;
- C₁-C₄-Alkylamino: Methylamino, Ethylamino, n-Propylamino, 1-Methylethylamino, n-Butylamino, 1-Methylpropylamino, 2-Methylpropylamino oder 1,1-Dimethylethylamino, vorzugsweise Methylamino oder Ethylamino;
- Di-(C₁-C₄-alkyl)amino: z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl) amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino 5 oder N-(1,1-Dimethylethyl)-N-(2-methylpropyl)amino, vorzugsweise Dimethylamino oder Diethylamino;
- (C₁-C₄-Alkyl)carbonyl: Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl oder 1,1-Dimethylethylcarbonyl, vorzugsweise Methylcarbonyl;
- (C₁-C₄-Alkoxy)carbonyl: Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl oder 1,1-Dimethylethoxycarbonyl, vorzugsweise Methoxycarbonyl, Ethoxycarbonyl oder 1,1-Dimethylethoxycarbonyl.

Heteroaryl steht vorzugsweise für einen 5- oder 6-gliedrigen aromatischen Heterocyclus mit einem Sauerstoff- und einem Schwefelatom oder ein 5- oder 6-gliedriger aromatischer Heterocyclus mit 1 bis 3 Heteroatomen, ausgewählt aus einer Gruppe bestehend aus 3 Stickstoffatomen und einem Sauerstoff- oder Schwefelatom: wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, l,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, insbesondere 2-Pridinyl, 3-Pyridinyl und 4-Pyridinyl.

Die 5- oder 6-gliedrigen Heteroarylringe tragen mindestens einen Substituenten, höchstens jedoch so viele, wie substituierbare Atome vorhanden sind.

Die Aryl- und Heteroarylringe Ar können gewünschtenfalls auch einen anellierten 5- oder 6-gliedrigen Ring tragen, der partiell ungesättigt oder aromatisch sein kann.

Im Hinblick auf die Verwendung der 2-Aroylcyclohexandione I als Herbizide oder zur Regulierung des Pflanzenwachstums bedeutet
- Ar: vorzugsweise Phenyl oder 5- oder 6-gliedriges Heteroaryl mit jeweils einem bis vier, insbesondere einem, zwei oder drei Substituenten, wobei jeder Substituent ausgewählt ist aus der Gruppe bestehend aus Halogen, Nitro, -N-N-Ph, -S(O)ₘR⁸, (C₁-C₄-Alkoxy) carbonyl, -N(R⁹)-COR¹⁰, -N (R⁹)-SO₂-R¹¹, -SO₂-N(R⁹)-R¹⁰, C₁-C₄-Alkyl, C₁-C₄-Alkoxy, C₁-C₄-Halogenalkyl und C₁-C₄-Halogenalkoxy;
oder eines der folgenden bicyclischen Ringsysteme: 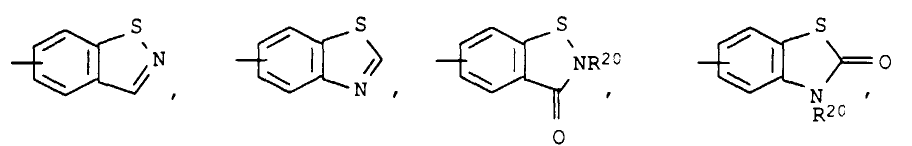 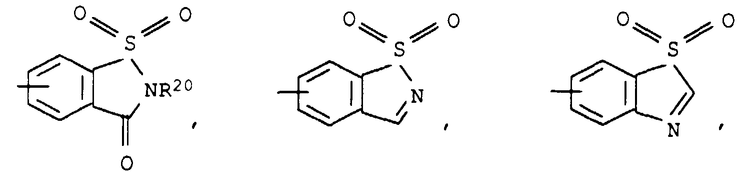 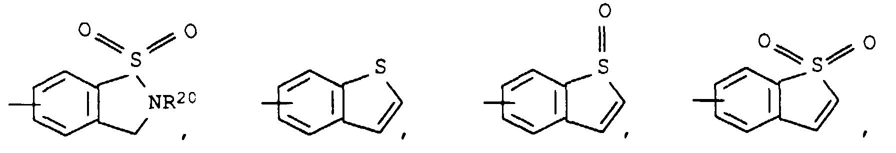 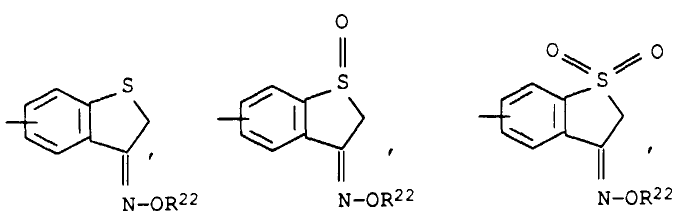 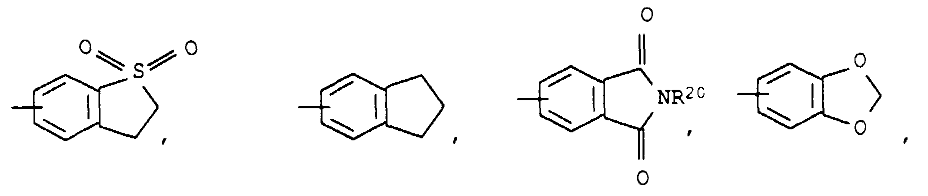 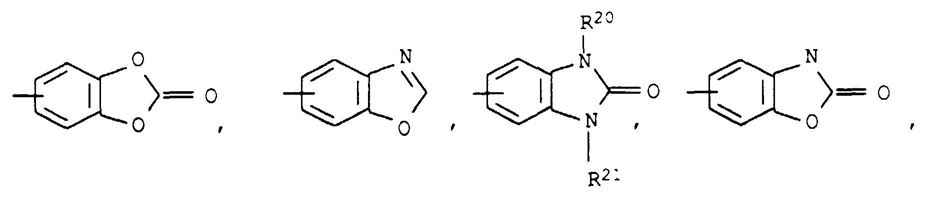 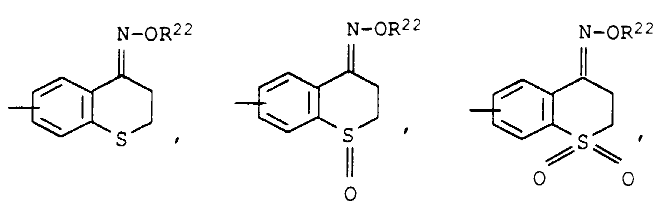 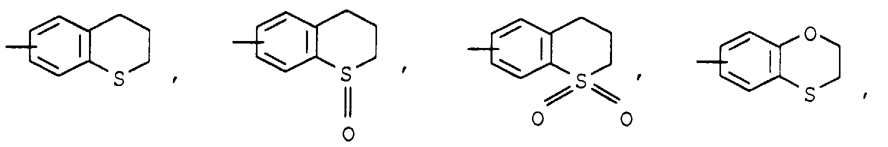 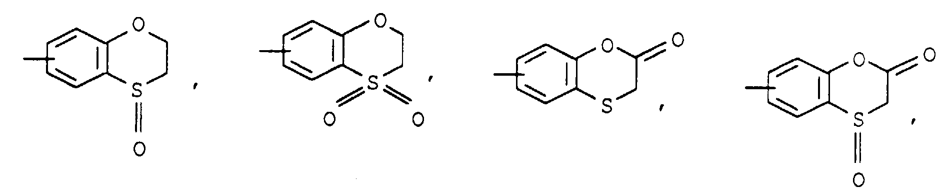 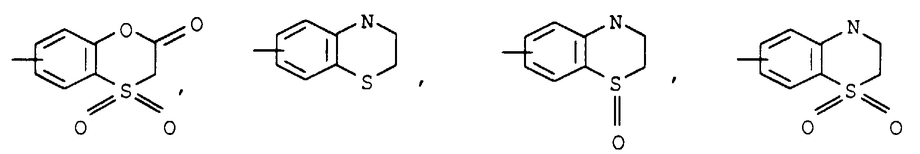 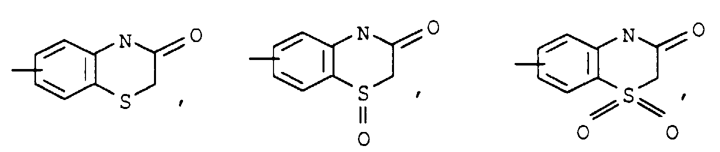 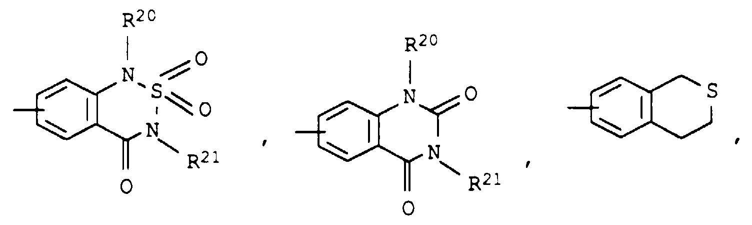 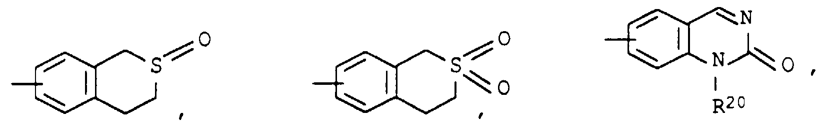 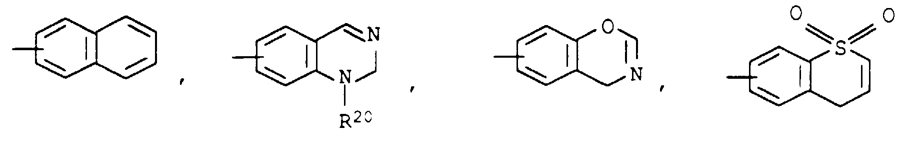 wobei R²⁰ und R²¹ jeweils vorzugsweise für Wasserstoff oder C₁-C₄-Alkyl stehen;

Ph steht vorzugsweise für Phenyl, das unsubstituiert sein oder gewünschtenfalls ein bis drei Substituenten tragen kann, ausgewählt aus der Gruppe bestehend aus Halogen, Cyano, Nitro, -S(O)ₙ-R²³, (C₁-C₄-Alkoxy)carbonyl, -SO₂-N(R²⁴)R²⁵, -N(R²⁴)-COR²⁵, -N(R²⁴)-SO₂-R²⁶, C₁-C₄-Alkyl, C₁-C₄-Alkoxy, C₁-C₄-Halogenalkyl und C₁-C₄-Halogenalkoxy;
m und n stehen unabhängig voneinander für 0, 1 oder 2;
R⁸ und R²³ stehen unabhängig voneinander vorzugsweise für C₁-C₄-Alkyl oder C₁-C₄-Halogenalkyl;
R⁹, R¹⁰, R²⁴ und R²⁵ stehen unabhängig voneinander vorzugsweise für Wasserstoff, C₁-C₄-Alkyl, Phenyl oder Phenyl, das ein bis drei Reste trägt, ausgewählt aus der Gruppe Halogen, C₁-C₄-Alkyl und C₁-C₄-Alkoxy;
R¹¹ und R²⁶ stehen unabhängig voneinander vorzugsweise für C₁-C₄-Alkyl oder C₁-C₄-Halogenalkyl;
R¹ bis R⁵ Wasserstoff und
R⁶ und R⁷ unabhängig voneinander vorzugsweise Methyl oder Ethyl oder zusammen Ethyliden, Propyliden oder 2,2-Di-(C₁-C₄-alkyl)-propyliden.

Ganz besonders bevorzugt wird diejenigen Verbindungen I, bei denen mindestens ein Substituent am Phenyl- oder Heteroarylring Ar einen Rest -N=N-Ph, -S(O)ₘ-R⁸, (C₁-C₄-Alkoxy)carbonyl, -N(R⁹)-SO₂-R¹¹, -SO₂-N(R⁹)R¹⁰, -N(R⁹)-COR¹⁰ oder C₁-C₄-Halogenalkyl bedeutet.

Aufgrund ihres sauren Charakters können die erfindungsgemäßen 2-Aroylcyclohexandione I basische Salze oder Enolester bilden, wobei es auf die Art des Salzes oder Esters im allgemeinen nicht ankommt.

Üblicherweise kommen diejenigen Salze und Ester in Betracht, bei denen die herbizide Wirkung im Vergleich zu der freien Verbindung I nicht negativ beeinträchtigt ist.

Als basische Salze eignen sich besonders diejenigen der Alkalimetalle, vorzugsweise Natrium- oder Kaliumsalze, der Erdalkalimetalle, vorzugsweise Calcium- und Magnesiumsalze, die der Übergangsmetalle, vorzugsweise Zink- oder Eisensalze, sowie Ammoniumsalze, bei denen das Ammoniumion gewünschtenfalls ein bis drei C₁-C₄-Alkyl-, Hydroxy-C₁-C₄-alkylsubstituenten und/oder einen Phenyl- oder Benzylsubstituenten tragen kann, vorzugsweise Diisopropylammonium-, Tetramethylammonium-, Tetrabutylammonium-, Trimethylbenzylammonium- und Trimethyl-(2-hydroxyethyl)ammoniumsalze, des weiteren Phosphoniumsalze, Sulfoniumsalze wie vorzugsweise Tri-(C₁-C₄-alkyl)sulfoniumsalze, und Sulfoxoniumsalze, wie vorzugsweise Tri- (C₁-C₄-alkyl)sulfoxoniumsalze.

Unter landwirtschaftlich brauchbaren Estern sind die Ester von
- C₁-C₁₀-Fettsäuren, insbesondere C₁-C₆-Alkylcarbonsäuren wie Methylcarbonsäure (Essigsäure), Ethylcarbonsäure (Propionsäure), n-Propylcarbonsäure (Buttersäure), 1-Methylethylcarbonsäure (Isobuttersäure), n-Butylcarbonsäure, 1-Methylpropylcarbonsäure, 2-Methylpropylcarbonsäure, 1,1-Dimethylethylcarbonsäure, n-Pentylcarbonsäure, 1-Methylbutylcarbonsäure, 2-Methylbutylcarbonsäure, 3-Methylbutylcarbonsäure, 1,1-Dimethylpropylcarbonsäure, 1,2-Dimethylpropylcarbonsäure, 2, 2-Dimethylpropylcarbonsäure, 1-Ethylpropylcarbonsäure, Benzoesäure sowie durch Halogen substituierte Benzoesäuren, n-Hexylcarbonsäure, 1-Methylpentylcarbonsäure, 2-Methylpentylcarbonsäure, 3-Methylpentylcarbonsäure, 4-Methylpentylcarbonsäure, 1,1-Dimethylbutylcarbonsäure, 1,2-Dimethylbutylcarbonsäure, 1,3-Dimethylbutylcarbonsäure, 2,2-Dimethylbutylcarbonsäure, 2,3-Dimethylbutylcarbonsäure, 3,3-Dimethylbutylcarbonsäure, 1-Ethylbutylcarbonsäure, 2-Ethylbutylcarbonsäure, 1,1,2-Trimethylpropylcarbonsäure, 1,2,2-Trimethylpropylcarbonsäure, 1-Ethyl-1-methylpropylcarbonsäure und 1-Ethyl-2-methylpropylcarbonsäure,
- C₁-C₁₀-Sulfonsäuren insbesondere C₁-C₆-Alkylsulfonsäuren wie Methylsulfonsäure, Ethylsulfonsäure, n-Propylsulfonsäure, 1-Methylethylsulfonsäure, n-Butylsulfonsäure, 1-Methylpropylsulfonsäure, 2-Methylpropylsulfonsäure, 1,1-Dimethylethylsulfonsäure, n-Pentylsulfonsäure, 1-Methylbutylsulfonsäure, 2-Methylbutylsulfonsäure, 3-Methylbutylsulfonsäure, 1,1-Dimethylpropylsulfonsäure, 1,2-Dimethylpropylsulfonsäure, 2,2-Dimethylpropylsulfonsäure, 1-Ethylpropylsulfonsäure, Benzolsulfonsäure sowie durch Halogen substituierte Benzolsulfonsäuren, n-Hexylsulfonsäure, 1-Methylpentylsulfonsäure, 2-Methylpentylsulfonsäure, 3-Methylpentylsulfonsäure, 4-Methylpentylsulfonsäure, 1,1-Dimethylbutylsulfonsäure, 1,2-Dimethylbutylsulfonsäure, 1,3-Dimethylbutylsulfonsäure, 2,2-Dimethylbutylsulfonsäure, 2,3-Dimethylbutylsulfonsäure, 3,3-Dimethylbutylsulfonsäure, 1-Ethylbutylsulfonsäure, 2-Ethylbutylsulfonsäure, 1,1,2-Trimethylpropylsulfonsäure, 1,2,2-Trimethylpropylsulfonsäure, 1-Ethyl-1-methylpropylsulfonsäure und 1-Ethyl-2-methylpropylsulfonsäure, und
- C₁-C₁₀-Phosphonsäuren, insbesondere C₁-C₆-Alkylphosphonsäuren wie Methylphosphonsäure, Ethylphosphonsäure, n-Propylphosphonsäure, 1-Methylethylphosphonsäure, n-Butylphosphonsäure, 1-Methylpropylphosphonsäure, 2-Methylpropylphosphonsäure, 1,1-Dimethylethylphosphonsäure, n-Pentylphosphonsäure, 1-Methylbutylphosphonsäure, 2-Methylbutylphosphonsäure, 3-Methylbutylphosphonsäure, 1,1-Dimethylpropylphosphonsäure, 1,2-Dimethylpropylphosphonsäure, 2,2-Dimethylpropylphosphonsäure, 1-Ethylpropylphosphonsäure, Benzolphosphonsäure sowie durch Halogen substituierte Benzolphosphonsäuren, n-Hexylphosphonsäure, 1-Methylpentylphosphonsäure, 2-Methylpentylphosphonsäure, 3-Methylpentylphosphonsäure, 4-Methylpentylphosphonsäure, 1,1-Dimethylbutylphosphonsäure, 1,2-Dimethylbutylphosphonsäure, 1,3-Dimethylbutylphosphonsäure, 2,2-Dimethylbutylphosphonsäure, 2,3-Dimethylbutylphosphonsäure, 3,3-Dimethylbutylphosphonsäure, 1-Ethylbutylphosphonsäure, 2-Ethylbutylphosphonsäure, 1,1,2-Trimethylpropylphosphonsäure, 1,2,2-Trimethylpropylphosphonsäure, 1-Ethyl-1-methylpropylphosphonsäure und 1-Ethyl-2-methylpropylphosphonsäure
zu verstehen.

Die 2-Aroylcyclohexandione I können in mehreren tautomeren Formen geschrieben werden, die alle von der Erfindung umfaßt werden:

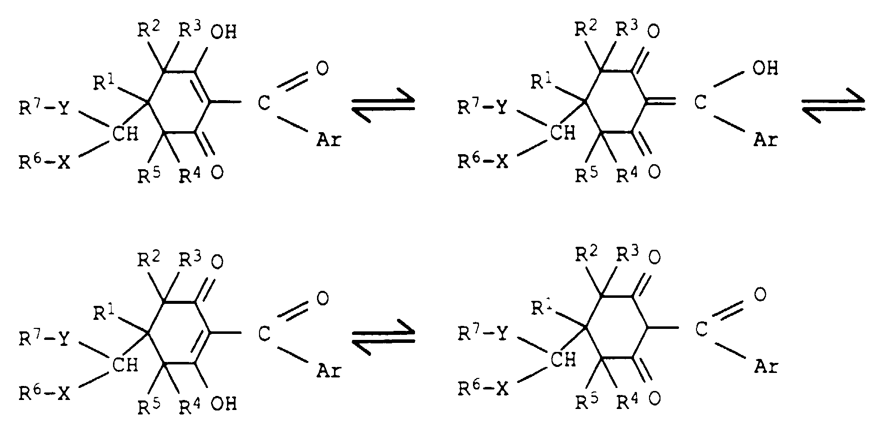

In der folgenden Tabelle 1 sind ganz besonders bevorzugte 2-Aroylcyclohexandione Ia (≙ I mit X und Y = Sauerstoff) aufgeführt:

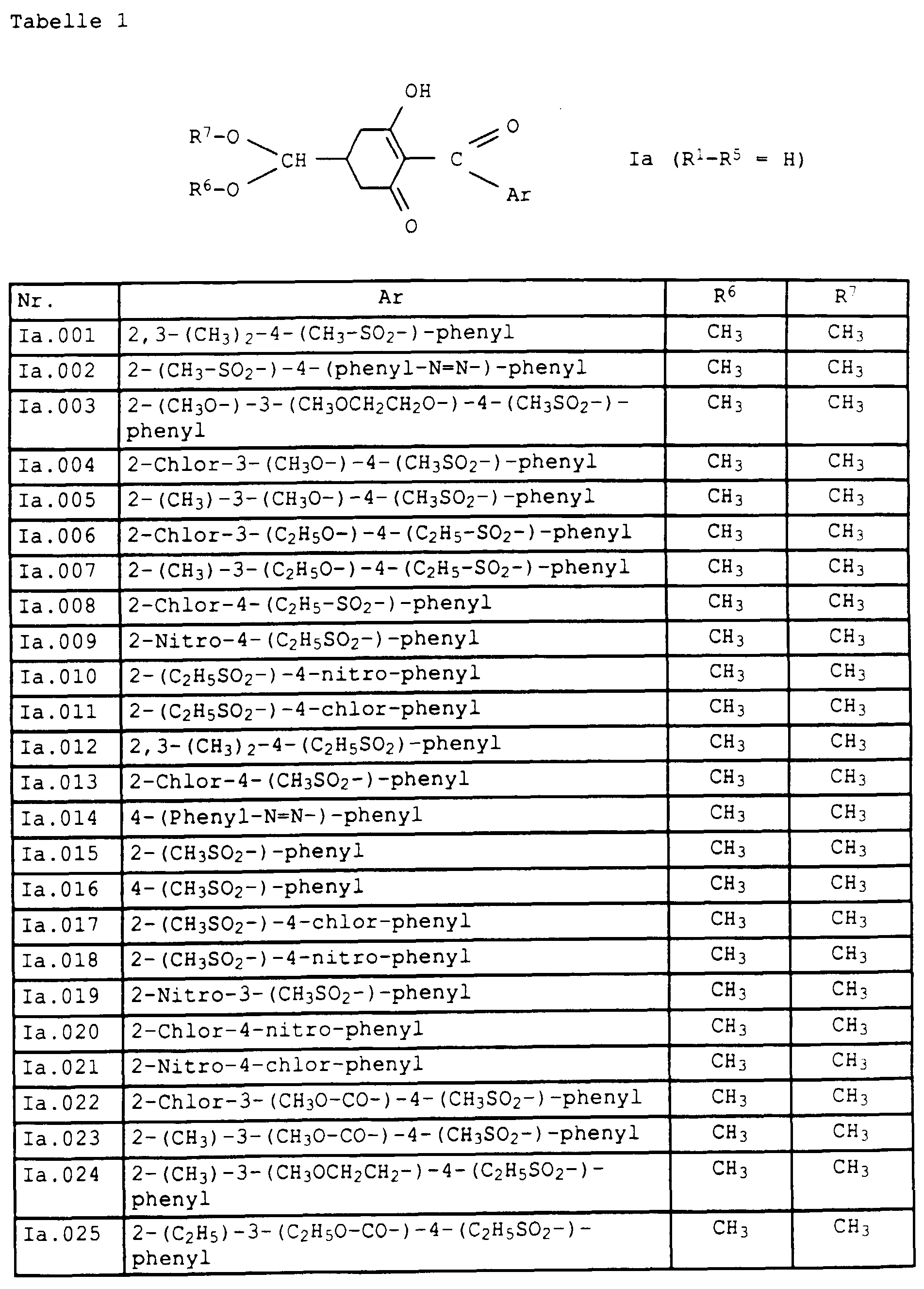

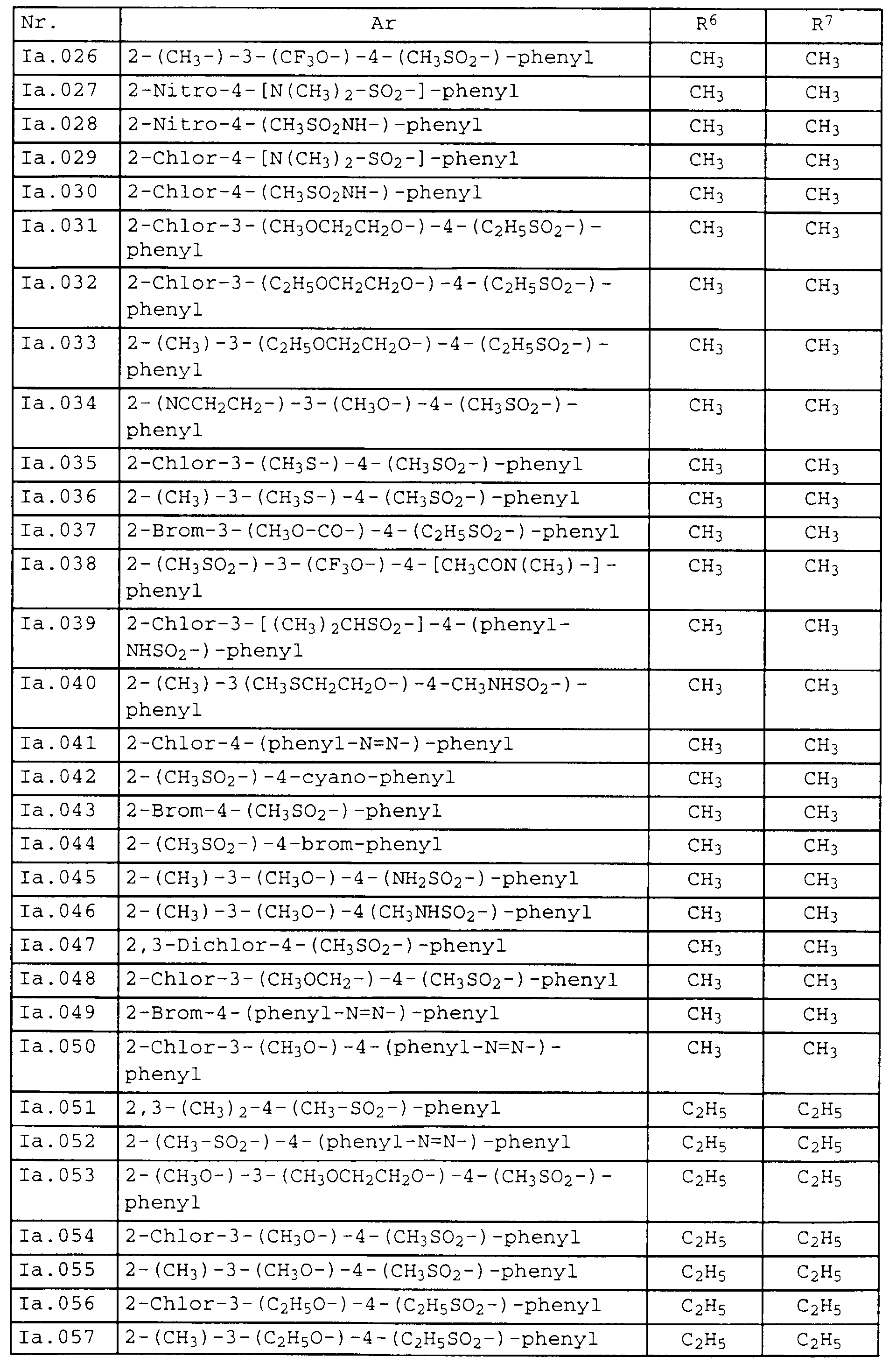

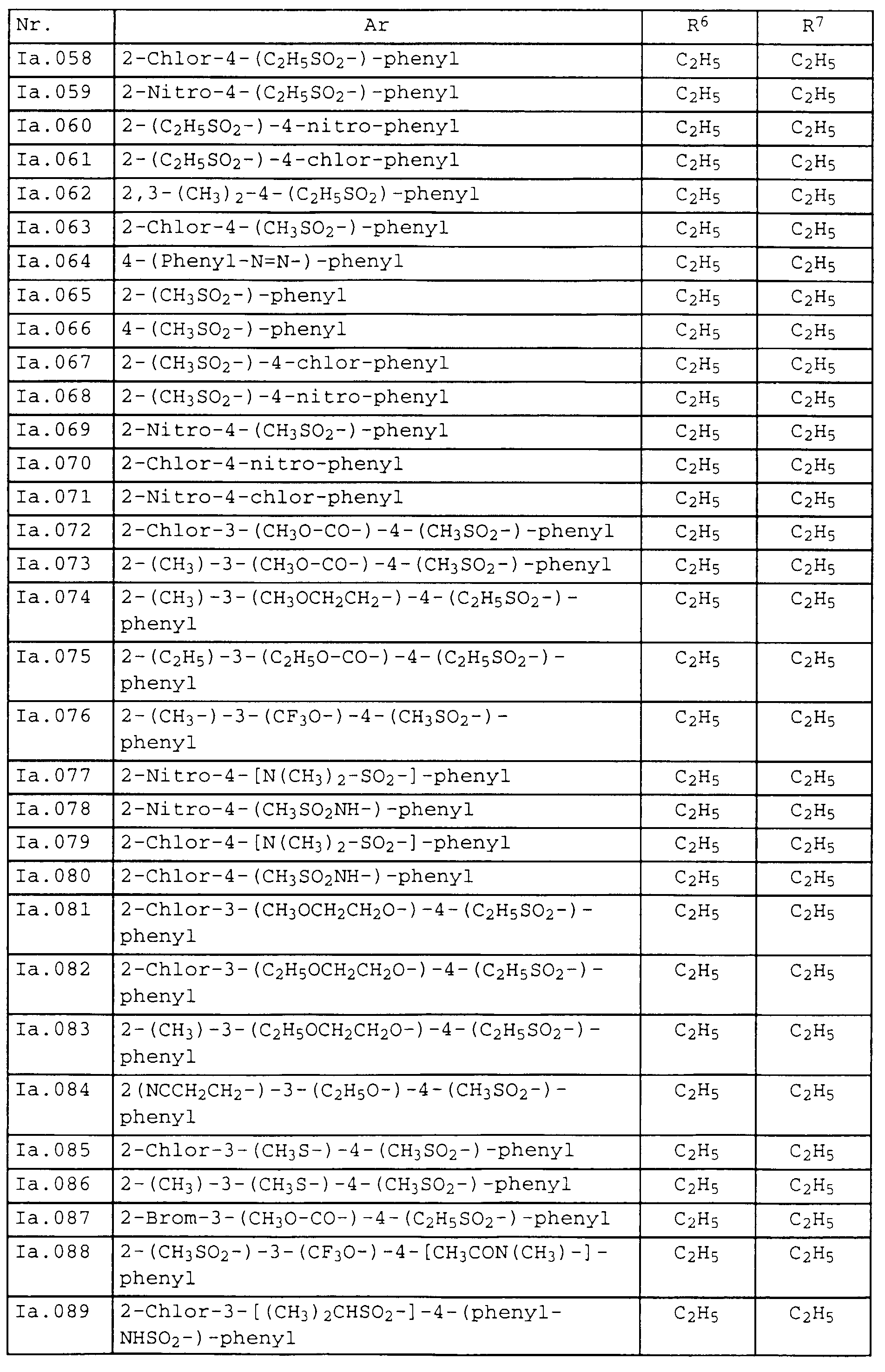

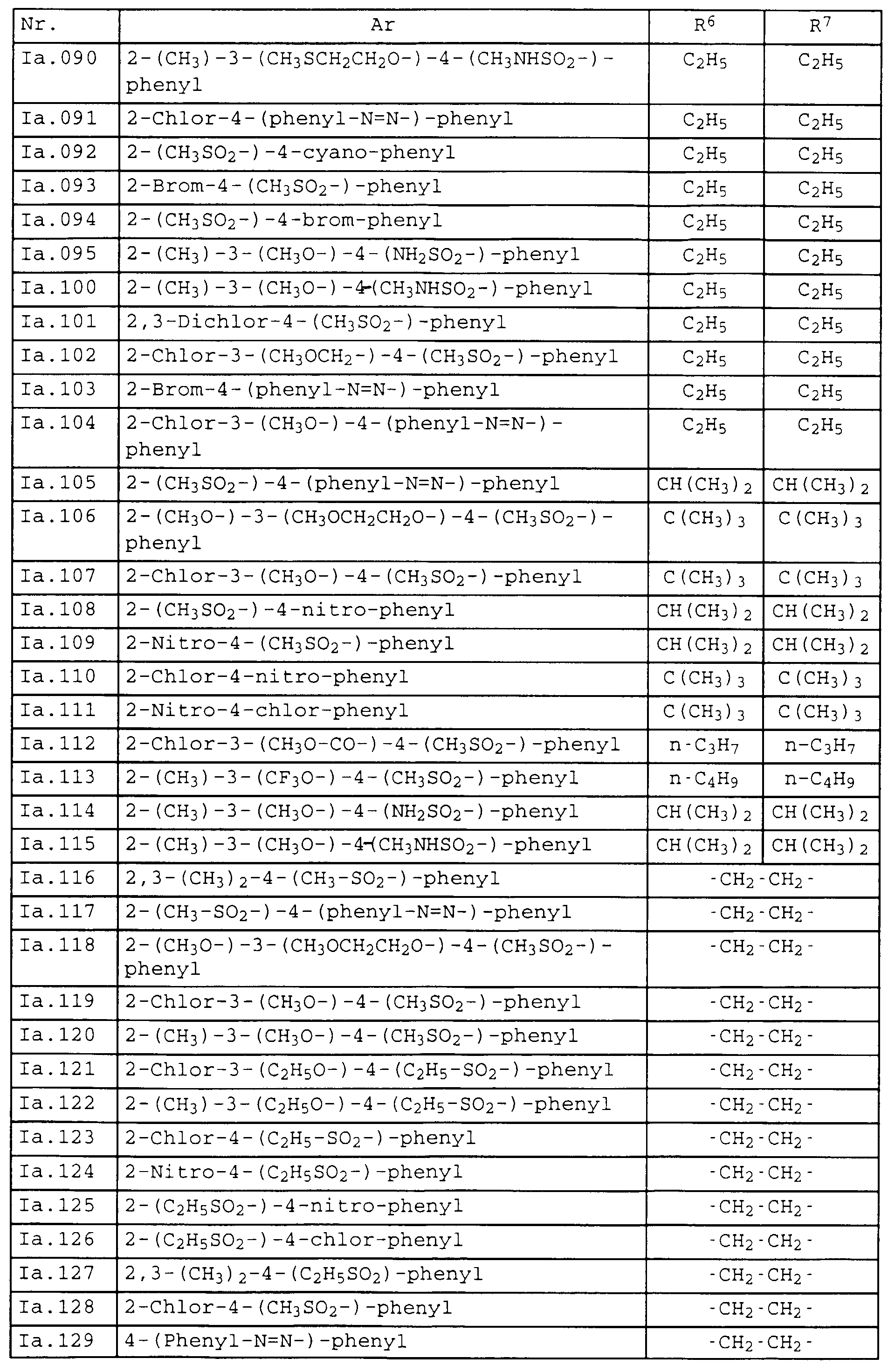

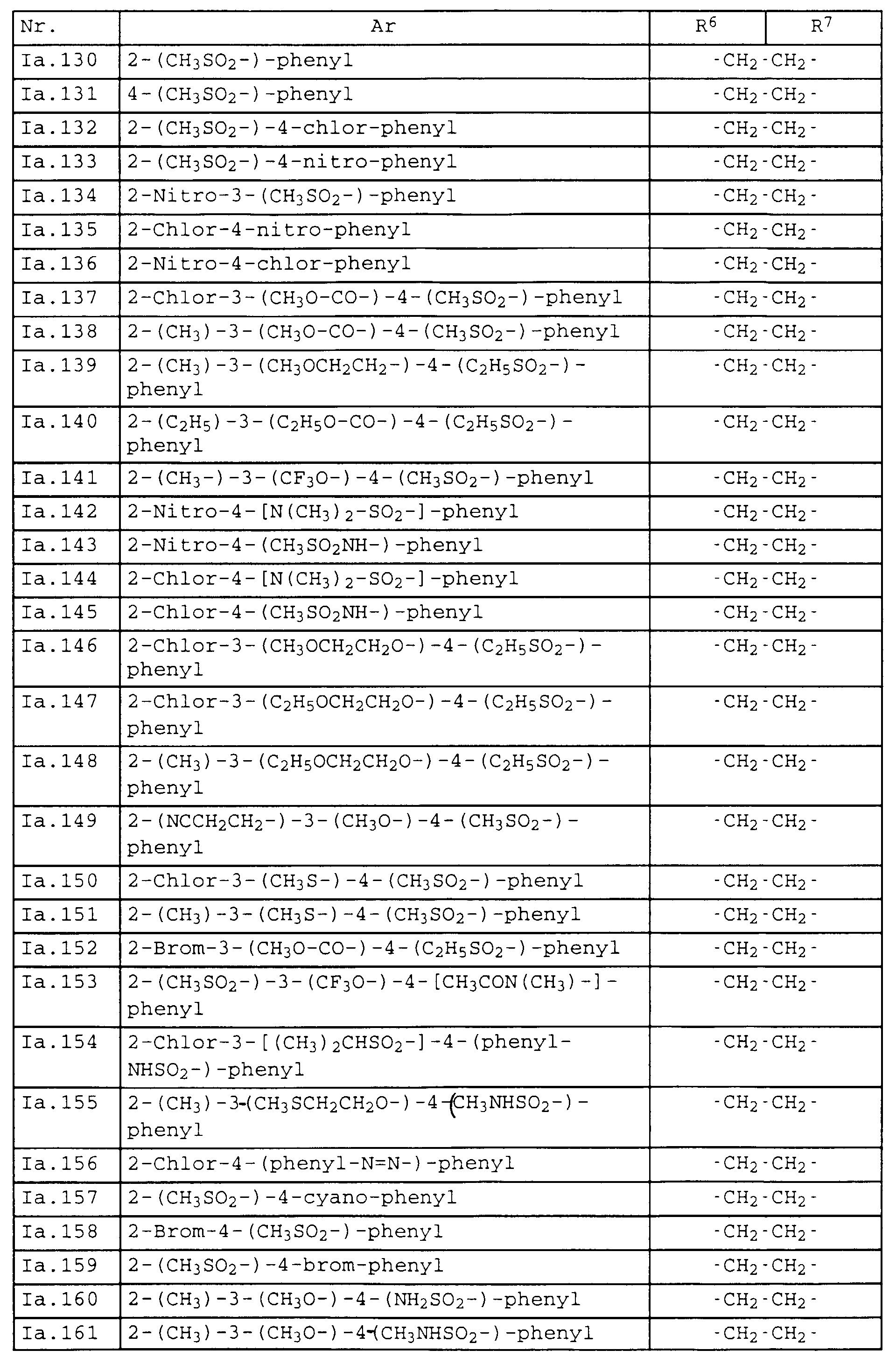

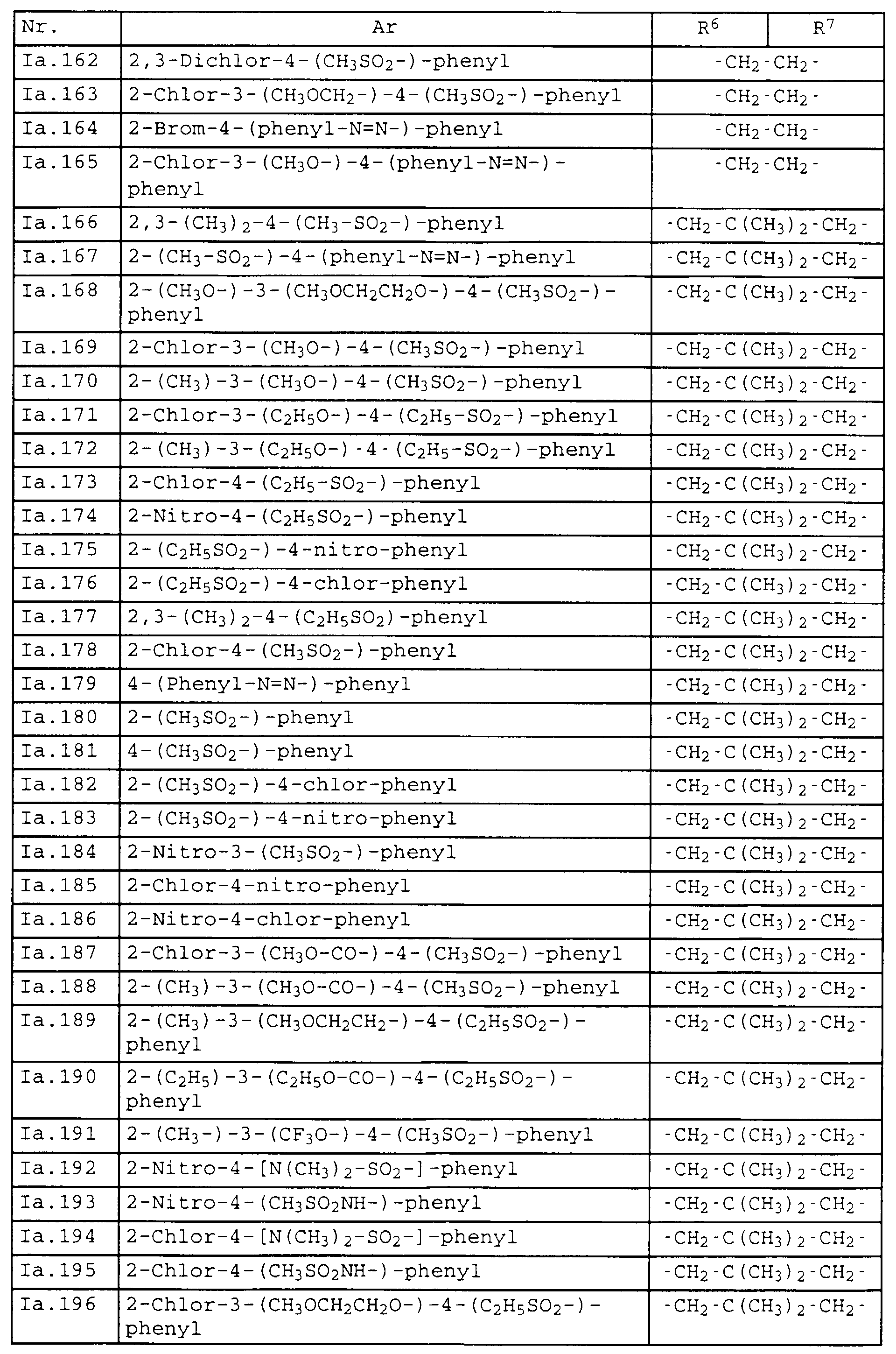

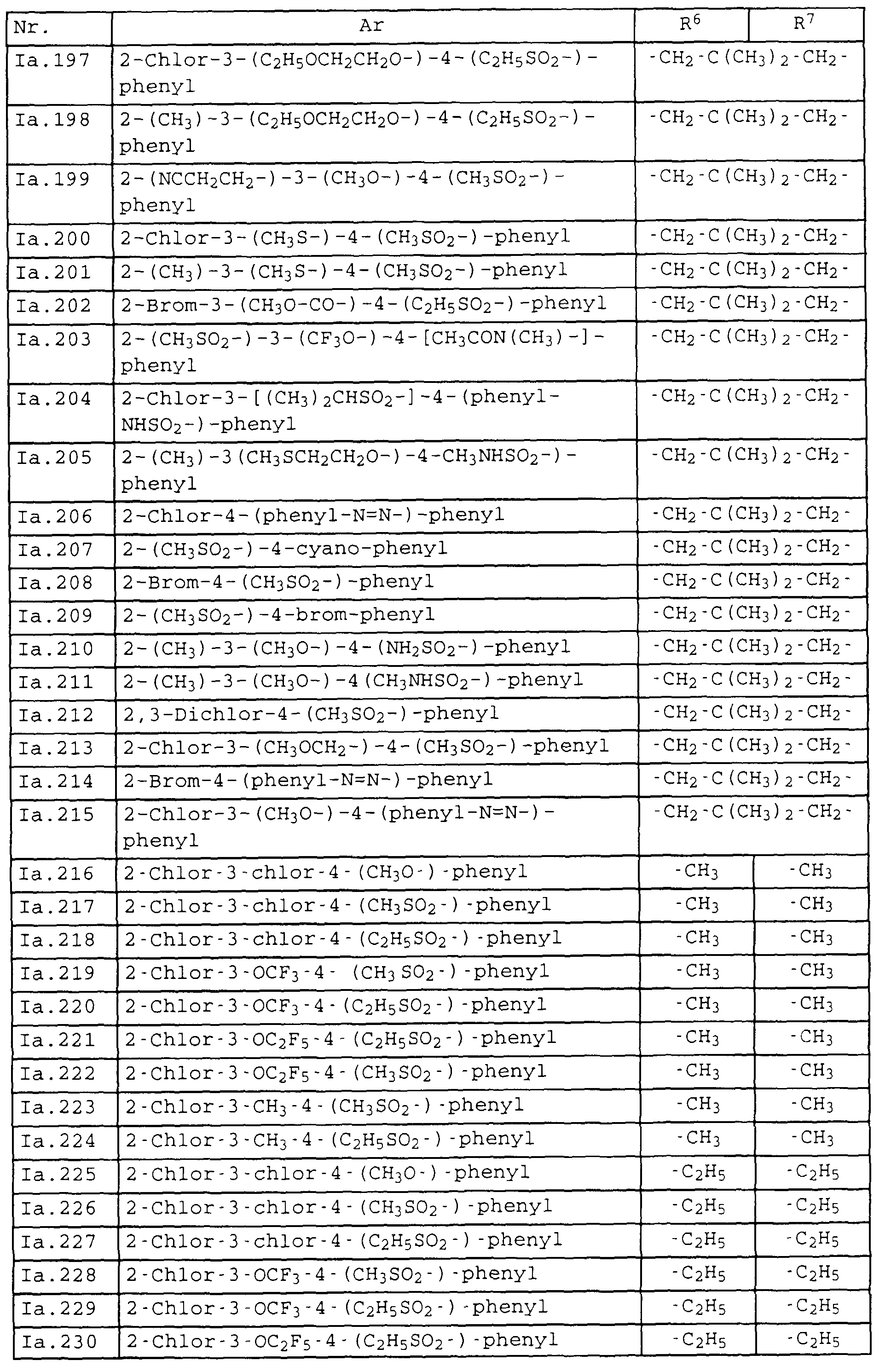

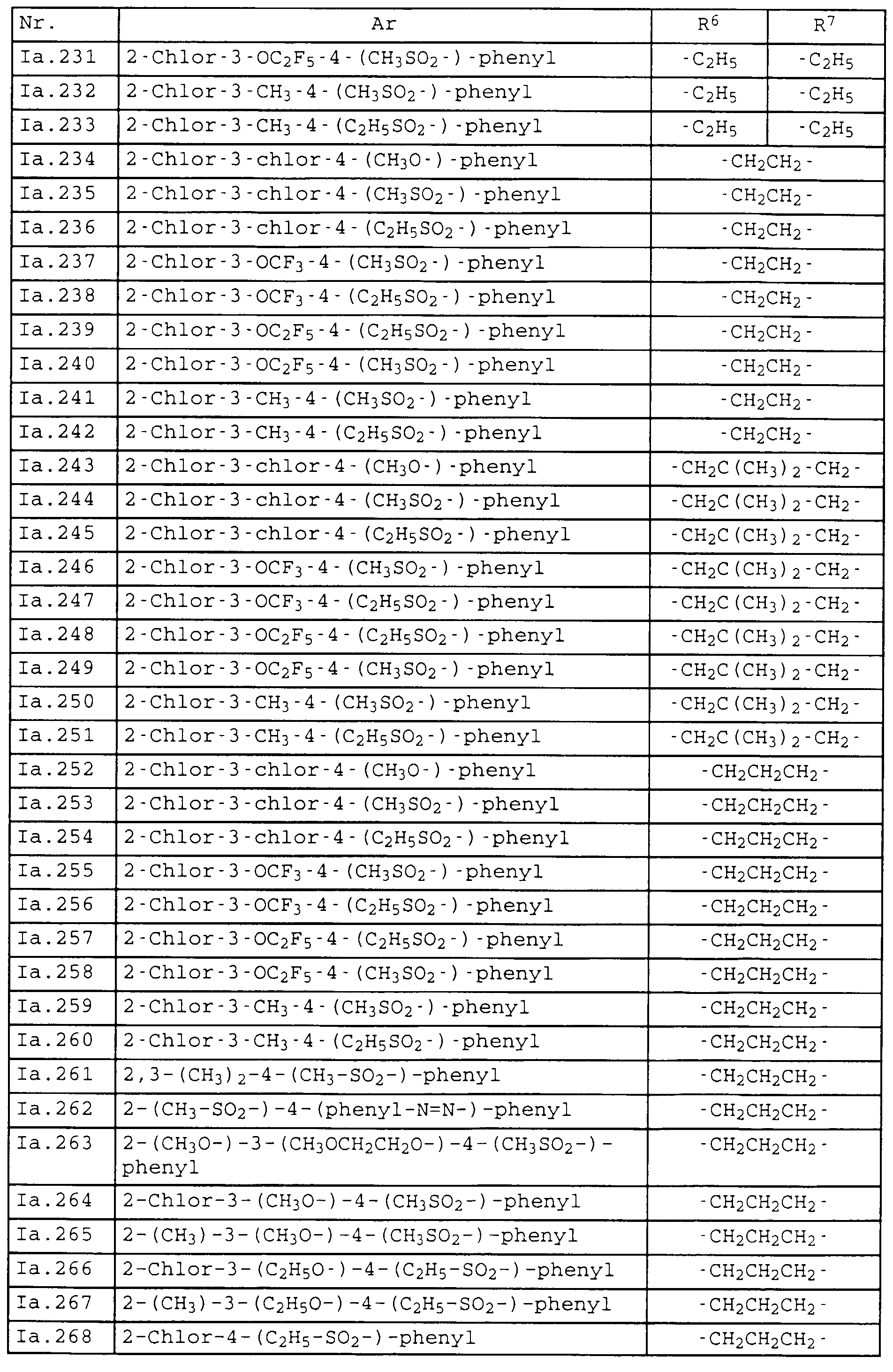

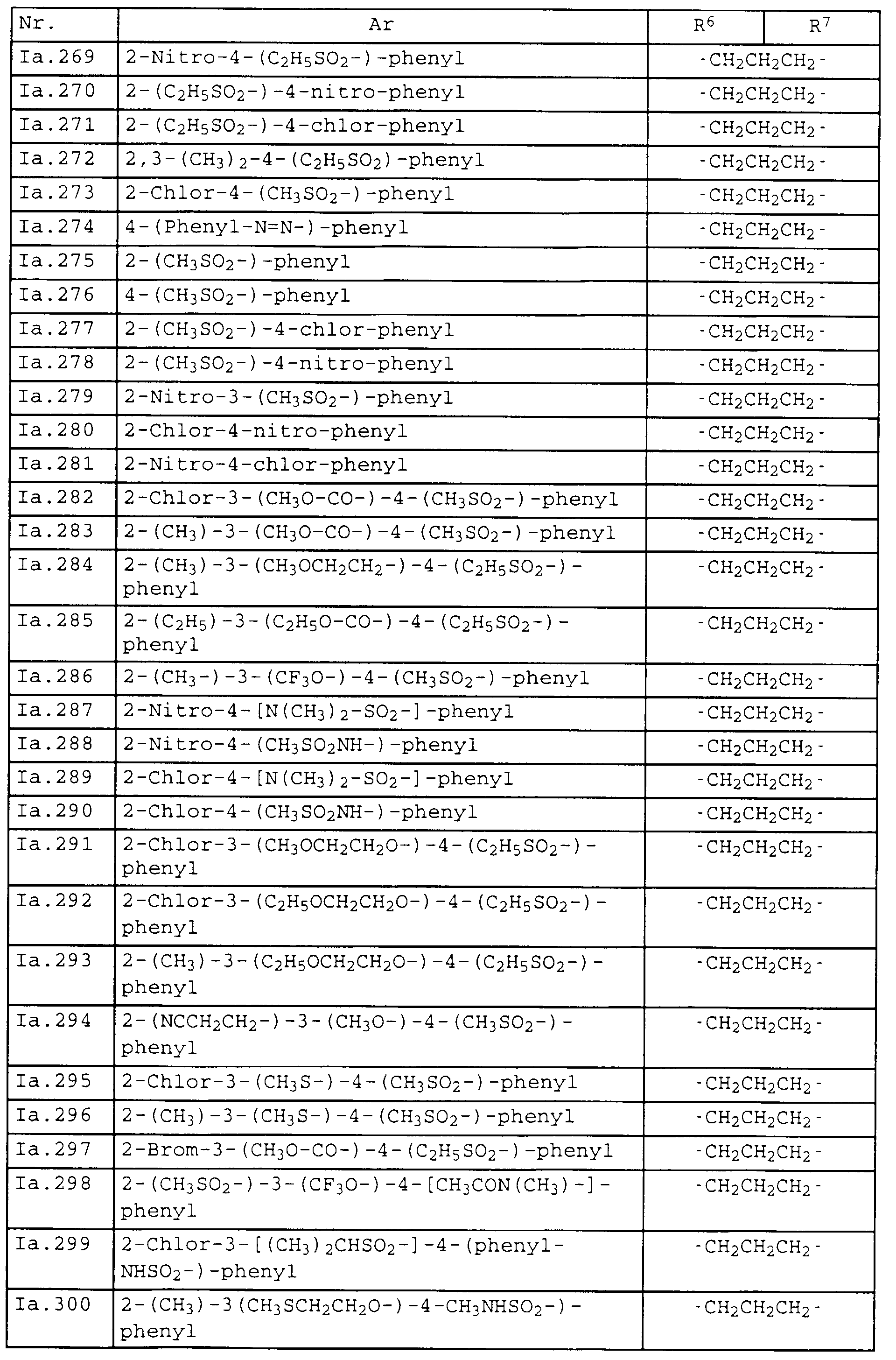

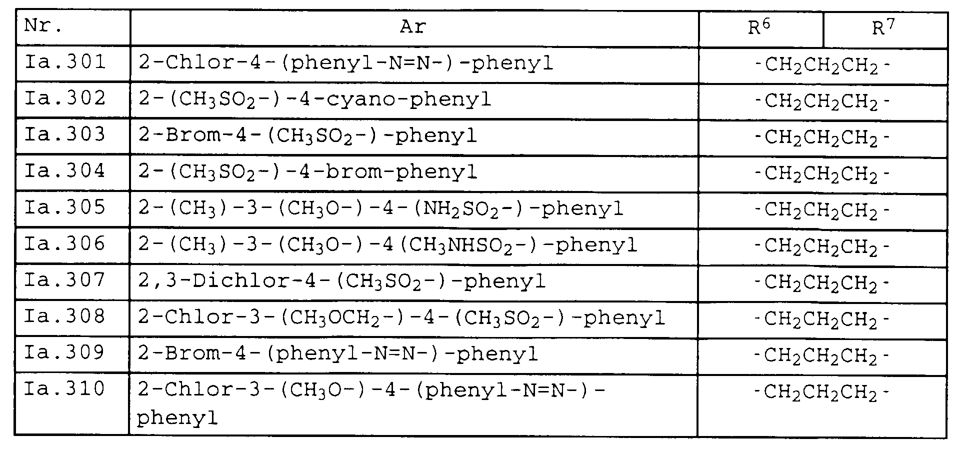

Die neuen 2-Aroylcyclohexandione I sind auf verschiedene Weise erhältlich, beispielsweise durch Umsetzung eines Cyclohexandions der Formel II mit einem Säurederivat der Formel III in Gegenwart einer Base (vgl. z.B. EP-A 186 118 und U.S. 4,695,673):

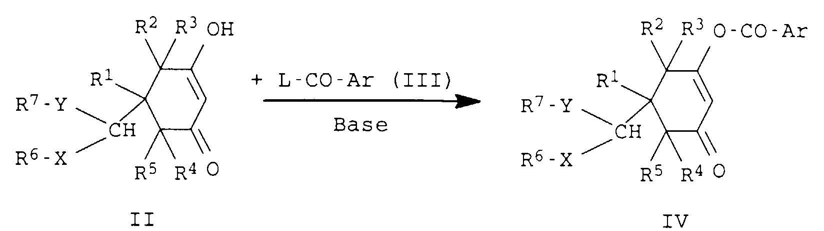

L steht für eine nukleophile Abgangsgruppe wie Chlorid, Bromid und Cyanid.

Die Reaktion wird in einem inerten Lösungsmittel zwischen 0°C und dem Siedepunkt des jeweiligen Lösungsmittels, vorzugsweise zwischen 0 und 20°C, durchgeführt.

Geeignete Lösungsmittel sind z.B. Chlorkohlenwasserstoffe wie Methylenchlorid, Chloroform und Dichlorethan, Ether wie Tetrahydrofuran, Dioxan und Methyltertiärbutylether, aromatische Kohlenwasserstoffe wie Benzol und Toluol, Ester wie Essigsäureethylester oder Amide wie Dimethylformamid.

Als Basen kommen beispielsweise tert. Amine wie Triethylamin, N-Methylmorpholin und Pyridin in Betracht.

Die Base wird vorteilhaft in etwa äquimolarer Menge, bezogen auf das Diketon II oder das Säurederivat III, eingesetzt.

Die Umsetzung kann auch in einem heterogenen System durchgeführt werden, wobei dann das Diketon II und die Base in wäßriger Lösung vorliegen, zu der das Säurederivat in einem mit Wasser nicht mischbaren Lösungsmittel, in Gegenwart eines Phasentransferkatalysators, gegeben wird.

Brauchbare Basen hierfür sind z.B. die Alkalimetall- und Erdalkalimetallhydroxide oder -carbonate wie Natriumhydroxid, Kaliumhydroxid, Natriumcarbonat, Kaliumcarbonat, Calciumcarbonat und Calciumhydroxid.

Beispiele für verwendbare Phasentransferkatalysatoren sind Ammonium-, Sulfonium- oder Phosphoniumsalze, wobei die Art des Anions von untergeordneter Bedeutung ist. Als zweckmäßig hat sich z.B. Benzyltrimethylammoniumhydroxid erwiesen.

Die erhaltenen Enolester der Formel IV werden dann in Gegenwart einer Cyanid-Quelle und einer Base in die gewünschten Verbindungen der Formel I umgelagert:

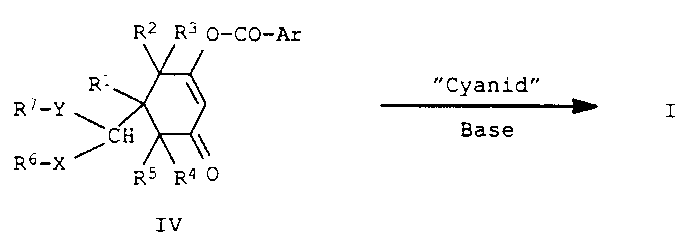

Die Umlagerung wird üblicherweise in einem inerten Lösungsmittel bei einer Temperatur zwischen 0°C und dem Siedepunkt des Lösungsmittels, vorzugsweise zwischen 20 und 40°C vorgenommen.

Geeignete Lösungsmittel sind z.B. Chlorkohlenwasserstoffe wie Methylenchlorid, Chloroform und Dichlorethan, Ether wie Tetrahydrofuran, Dioxan und Methyltertiärbutylether, aromatische Kohlenwasserstoffe wie Benzol und Toluol, Nitrile wie Acetonitril, Ester wie Essigsäureethylester, Ketone wie Aceton und Methylethylketon oder Amide wie Dimethylformamid.

Als Cyanidquellen haben sich z.B. Alkalimetallcyanide wie Natriumcyanid und Kaliumcyanid, Cyanhydrine wie Acetoncyanhydrin und Trialkylsilylcyanide wie Trimethylsilylcyanid als geeignet erwiesen.

Normalerweise ist eine katalytische Menge an Cyanid, etwa zwischen 1 und 10 Mol%, bezogen auf den Enolester IV, ausreichend.

Brauchbare Basen sind z.B. Trialkylamine wie Triethylamin, Trialkanolamine wie Triethanolamin und Pyridin oder anorganische Basen wie Alkalimetallcarbonate und -phosphate.

Es hat sich als vorteilhaft erwiesen, die Base in einem Überschuß von 100 bis 400 Mol%, bezogen auf den Enolester IV, zuzusetzen.

Aus den nach diesem Verfahren erhaltenen Reaktionsgemischen können die 2-Aroylcyclohexandione I mittels üblicher Aufarbeitungsmethoden isoliert werden, beispielsweise durch Extraktion oder durch Kristallisation.

Besondere Bedingungen bezüglich des Drucks sind bei der Herstellung der Verbindungen I nicht zu beachten; im allgemeinen arbeitet man daher bei Normaldruck oder unter dem Eigendruck des jeweiligen Verdünnungsmittels.

Die Diketone der Formel II sind aus der EP-A 233 568 bekannt oder können auf die dort beschriebene Weise hergestellt werden.

Die benötigten Säurederivate III sind bekannt oder können nach an sich bekannten Methoden erhalten werden {vgl. z.B. The Chemistry of Carboxylic Acids and Esters, S. Patai, editor, J. Wiley and Sons, New York, N.Y.(1969); Survey of Organic Synthesis, C.A. Buehler and D.F. Pearson, J. Wiley and Sons, (1970); Reagents for Organic Synthesis, Vol. I, L.F. Fieser and M. Fieser, S. 767-769 (1967)}.

Alkalimetallsalze der Verbindungen I können durch Behandeln von I mit Natrium- oder Kaliumhydroxid oder -alkoholat in wäßriger Lösung oder in einem organischen Lösungsmittel wie Methanol, Ethanol, Aceton, Toluol erhalten werden.

Andere Metallsalze, z. B. die Mangan-, Kupfer-, Zink-, Eisen-, Calcium-, Magnesium- und Bariumsalze, können aus den Natriumsalzen in üblicher Weise hergestellt werden, ebenso Ammonium-, Phosphonium-, Sulfonium- oder Sulfoxoniumsalze mittels Ammoniak, Phosphonium-, Sulfonium- oder Sulfoxoniumhydroxiden.

Die Ester der Verbindungen I sind ebenfalls in üblicher Weise erhältlich (vgl. z.B. EP-A 102 823 und EP-A 136 702).

Die 2-Aroylcyclohexandione I, deren Salze und Ester - bzw. die diese Verbindungen enthaltenden Mittel - können in Kulturen wie Weizen, Reis, Mais, Soja und Baumwolle Unkräuter und Schadgräser sehr gut bekämpfen, ohne die Kulturpflanzen zu schädigen. Dieser Effekt tritt vor allem bei niedrigen Aufwandmengen auf.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew. %, vorzugsweise zwischen 0,5 und 90 Gew. %, Wirkstoff. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können weiterhin beispielsweise wie folgt formuliert werden:
I. 20 Gewichtsteile der Verbindung Nr. 5 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. 9 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. 1 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.
V. 3 Gewichtsteile des Wirkstoffs Nr. 4 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. 7 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 2,0 kg/ha aktive Substanz (a.S.).

In Anbetracht der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus lassen sich die Verbindungen I in Kulturen, die durch Züchtung und/oder mittels gentechnischer Methoden gegen die Wirkung von I weitgehend resistent gemacht wurden, einsetzen.

Die Verbindungen der Formel I können weiterhin die verschiedenen Entwicklungsstadien einer Pflanze beeinflussen und deshalb als Wachstumsregulatoren eingesetzt werden. Die Wirkungsvielfalt der Pflanzenwachstumsregulatoren ist vor allem abhängig
a) von der Pflanzenart und -sorte,
b) von dem Zeitpunkt der Applikation, bezogen auf das Entwicklungsstadium der Pflanze, und von der Jahreszeit,
c) von dem Applikationsort und -verfahren (z. B. Samenbeize, Bodenbehandlung, Blattapplikation oder Stamminjektion bei Bäumen),
d) von klimatischen Faktoren (z. B. Temperatur, Niederschlagsmenge, außerdem auch Tageslänge und Lichtintensität),
e) von der Bodenbeschaffenheit (einschließlich Düngung),
f) von der Formulierung bzw. Anwendungsform des Wirkstoffs und
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt:
A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern an Straßenrändern, Hekken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.
   Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknikens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchte zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
   - die Öffnungsweite der Stomata reduziert wird,
   - eine dickere Epidermis und Cuticula ausgebildet werden,
   - die Durchwurzelung des Bodens verbessert wird und
   - das Mikroklima im Pflanzenbestand durch einen kompakteren Wuchs günstig beeinflußt wird.

Besonders gut eignen sich die Verbindungen I zur Halmverkürzung von Kulturpflanzen wie Gerste, Raps und Weizen.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt, zugeführt werden. Die Herstellung der Mittel erfolgt dabei analog zu der von Herbiziden (s.o.).

Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Zur Verbreiterung des Wirkungsspektrums und zur Erzielung synergistischer Effekte können die 2-Aroylcyclohexandione I mit zahlreichen Vertretern anderer herbizider oder wachstumsregulierender Wirkstoffgruppen gemischt und gemeinsam ausgebracht werden. Beispielsweise kommen als herbizide Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht. Bei Wachstumsregulatoren kommen insbesondere Chlormequatchlorid, Ethephon und Mepiquatchlorid in Frage.

Außerdem kann es von Nutzen sein, die Verbindungen I allein oder in Kombination mit anderen Herbiziden oder Wachstumsregulatoren auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiel

### 2-(2-Chlor-4-methylsulfonyl-benzoyl)-5-dimethoxymethyl-1,3-cyclohexandion

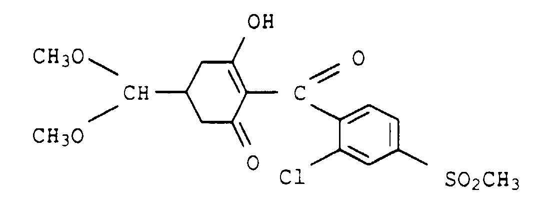

Eine Lösung von 1,7 g (4,2 mmol) 5-Dimethoxymethyl-3-oxo-1-cyclohexen-1-yl)-2-chlor-4-methylsulfoyl-benzoat und 0,2 g (2,3 mmol) Acetoncyanhydrin in 70 ml Acetonitril wurde bei ca. 20°C mit 2,2 g (22 mmol) Triethylamin in 10 ml Acetonitril gerührt und tropfenweise mit 30 ml einer 3 %igen wäßrigen Salzsäurelösung versetzt. Anschließend extrahierte man das Reaktionsgemisch mit Methyl-tert.-butylether und wusch viermal mit 5 %iger wäßriger Kaliumcarbonat-Lösung. Die vereinigten wäßrigen Phasen wurden bei 10 - 20°C mit Salzsäure angesäuert und dann zweimal mit Methyltert.-butylether extrahierte. Die vereinigten Etherphasen wurden getrocknet, filtriert und dann chromatographisch an Kieselgel gereinigt. Ausbeute: 41 %.

### Vorstufe:

### 5-Dimethoxymethyl-3-oxo-1-cyclohexen-1-yl)-2-chlor-4-methylsulfonyl-benzoat

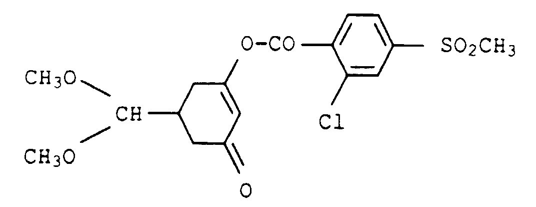

Zu einer Mischung aus 1,0 g (5,4 mmol) 5-Dimethoxymethyl-1,3-cyclohexandion, 0,54 g (5,4 mmol) Triethylamin und 20 ml Tetrahydrofuran wurden bei 0°C 1,4 g (5,4 mmol) 2-Chlor-4-methylsulfonyl-benzoylchlorid in 10 ml Tetrahydrofuran getropft. Anschließend rührte man eine Stunde bei 0°C und weitere 2 Stunden bei ca. 20°C, wonach 80 ml Methylchlorid in das Reaktionsgemisch gegeben wurden. Man wusch die organische Phase einmal mit Wasser, einmal mit halbkonzentrierter wäßriger Natriumcarbonatlösung und dann noch zweimal mit Wasser. Nach dieser Reinigung wurde die organische Phase getrocknet, filtriert und schließlich eingeengt. Ausbeute: 79 %.

In den folgenden Tabellen 2 und 3 sind noch weitere 2-Aroylcyclohexandione I aufgeführt, die auf die gleiche Weise hergestellt wurden oder herstellbar sind:

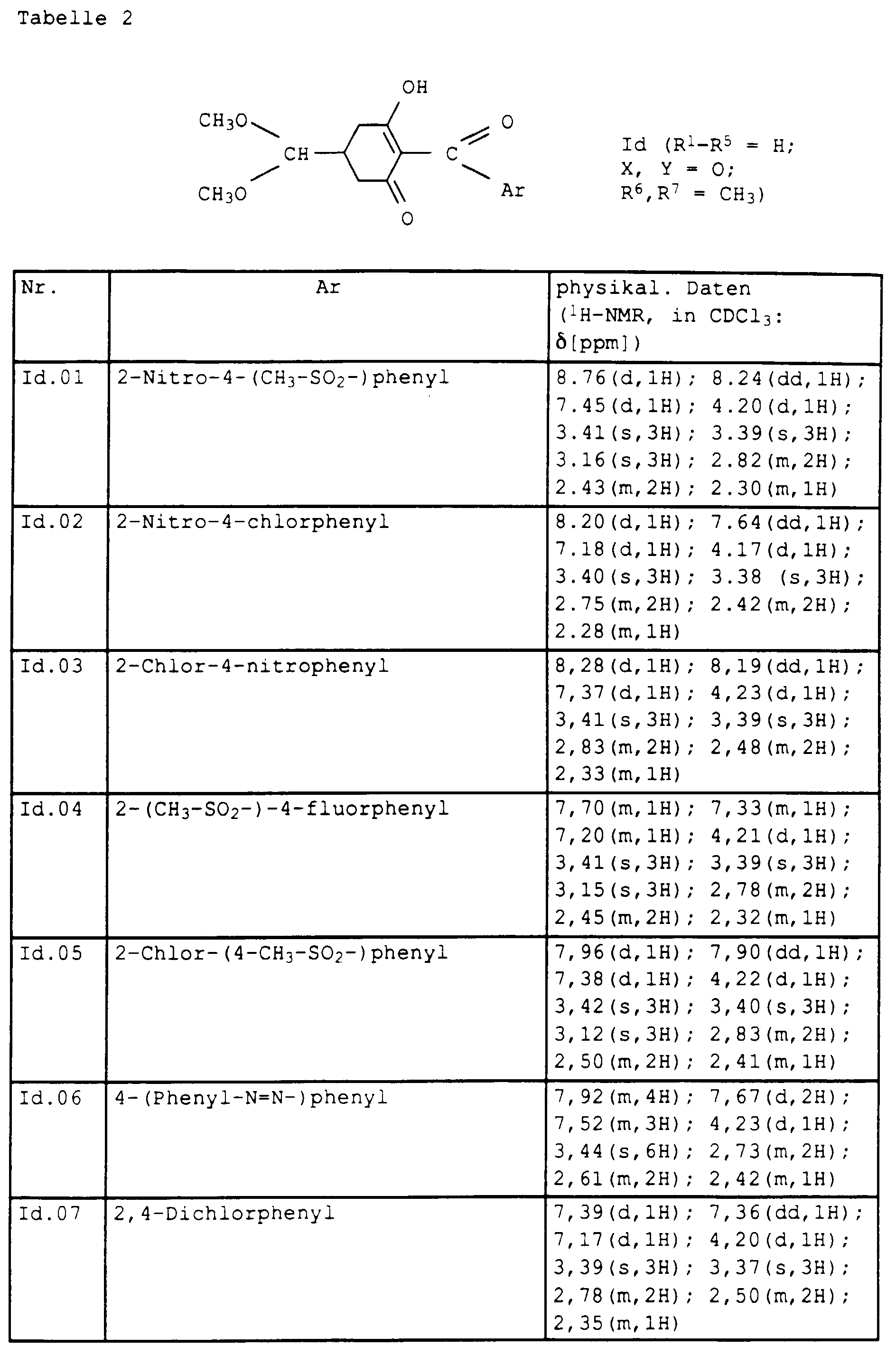

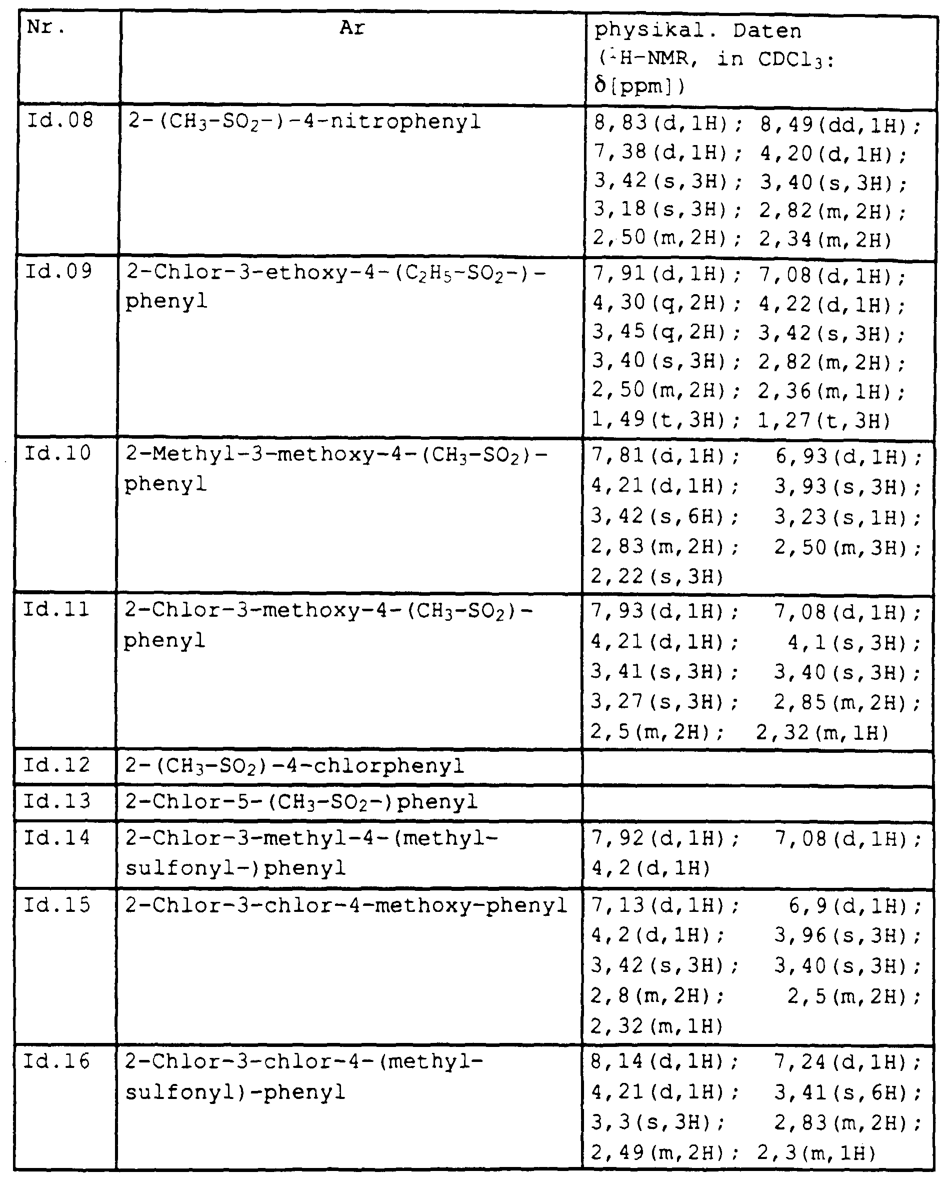

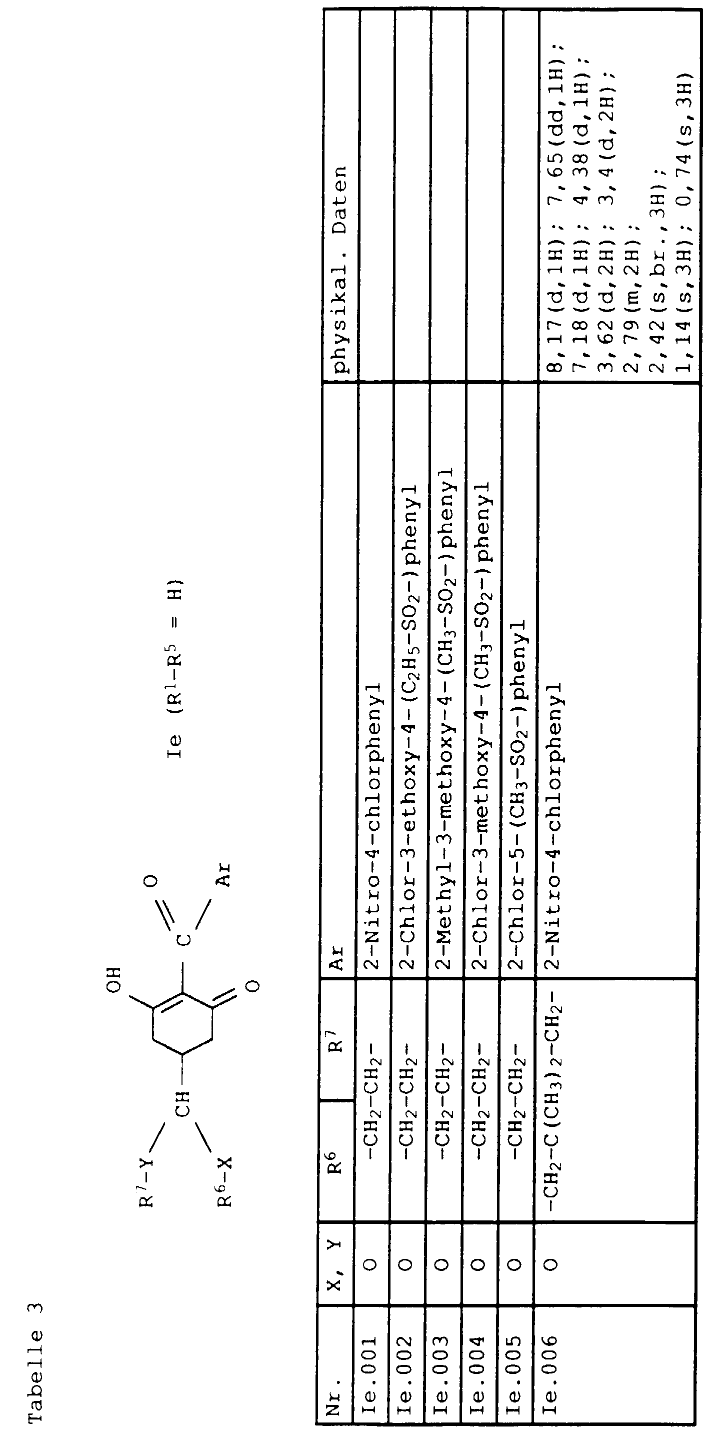

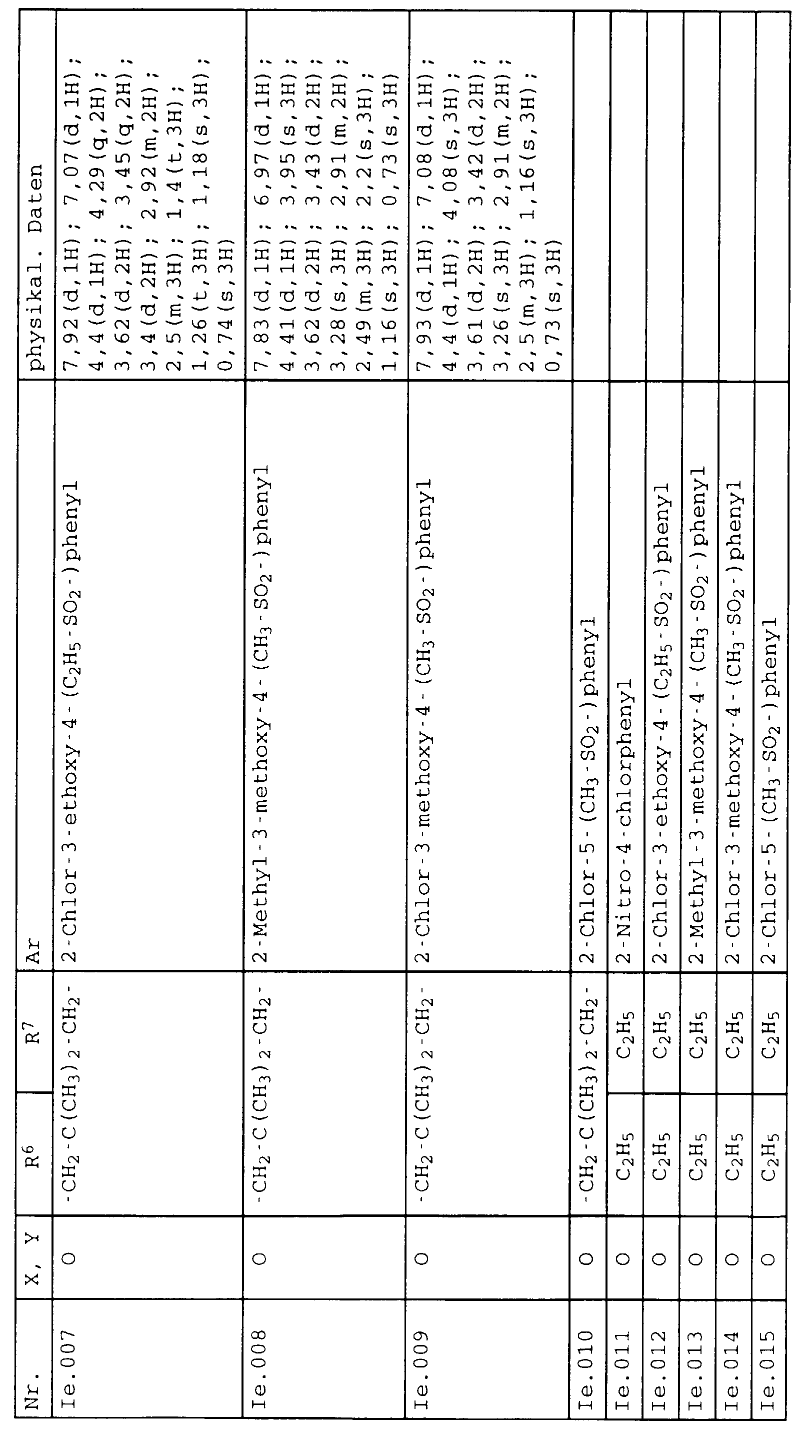

### Anwendungsbeispiele

Die herbizide und wachstumsregulatorische Wirkung der 2-Aroylcyclohexandione der Formel I ließ sich durch Gewächshausversuche zeigen:

Als Kulturgefäße dienten Plastikblumentöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung beträgt 0,25 bzw. 0,125 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die wachstumsregulierende Wirkung wurde durch Längenmessung bestimmt. Bei Versuchsende wurden die Wuchshöhen der behandelten Pflanzen gemessen und zur Wuchshöhe von nicht behandelten Pflanzen in Beziehung gesetzt.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | lambsquarters (goosefoot) |
| Echinochloa crus-galli | Hühnerhirse | barnyardgrass |
| Setaria viridis | Grüne Borstenhirse | green foxtail |
| Solanum nigrum | Schwarzer Nachtschatten | black nightshade |
| Triticum aestivum | Winterweizen | winter wheat |
| Veronica spp. | Ehrenpreisarten | speedwell |
| Zea mays | Mais | Indian corn |

Das Ergebnis zeigte, daß mit den Verbindungen Nr. 05 und 09 unerwünschte Pflanzen sehr gut bekämpft werden können.

## Patentansprüche

1. 2-Aroylcyclohexandione der Formel I 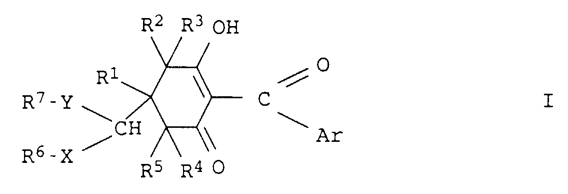 in der die Variablen folgende Bedeutung haben:
X und Y Sauerstoff;
Ar
Phenyl oder einen 5- oder 6-gliedrigen Heteroarylring, wobei der Phenyl- oder Heteroarylring mindestens einen, höchstens jedoch vier Substituenten trägt, jeweils ausgewählt aus der Gruppe bestehend aus Halogen, Cyano, Nitro, -N=N-Ph, (C₁-C₄-Alkoxy) carbonyl, -N(R⁹)-COR¹⁰, -N (R⁹)-SO₂-R¹¹, -SO₂-N(R⁹)R¹⁰, -S(O)ₘ-R⁸, C₁-C₄-Alkyl, C₁-C₄-Alkoxy, C₁-C₄-Halogenalkyl und C₁-C₄-Halogenalkoxy, wobei die vier letztgenannten Reste ihrerseits ein oder zwei der folgenden Substituenten tragen können: C₁-C₄-Alkoxy, C₁-C₄-Alkylthio und/oder Cyano,
und wobei zwei benachbarte C-Atome des Phenyl- oder Heteroarylrings auch über eine Kette -C(R¹²)=C(R¹³)-C(R¹⁴)=C(R¹⁵)-, -Z¹-C(R¹²)=N-, -Z¹-N=C(R¹²)-, -Z¹-C(R¹²)=C(R¹³)-, -Z¹-C(R¹²)=C(R¹³)-C(R¹⁴, R¹⁵)-, -Z¹-C(R¹², R¹³)-C(R¹⁴, R¹⁵)-, -Z¹-C(R¹², R¹³)-C(R¹⁴, R¹⁵)-Z²-, -C(R¹², R¹³)-Z¹-C(R¹⁴, R¹⁵)-C(R¹⁶, R¹⁷)-, -Z¹-N(R²⁰)-Z²-, -Z¹-Z²-N(R²⁰)-, -Z¹-C(R¹², R¹³)-Z²-N(R²⁰)-, -Z¹-N(R²⁰)-Z²-N(R²¹)-, -N(R²¹)-Z¹-Z²-, -N(R²⁰)-Z¹-N=C(R¹²)-, -Z¹-C(R¹², R¹³)-C(=NOR²²)-, -Z¹-C(R¹², R¹³)-C(R¹⁴, R¹⁵)-C(=NOR²²)- oder Z¹-Z²-Z¹ verbrückt sein können, wobei
Z¹ und Z² unabhängig voneinander
Sauerstoff, Schwefel, -SO-, -SO₂-, -CO-, -C(R¹⁸, R¹⁹)- oder-N(R²⁰)- bedeuten und wobei
R¹² bis R¹⁹ unabhängig voneinander für
Wasserstoff, Halogen, Hydroxyl, C₁-C₄-Alkyl, C₁-C₄-Alkoxy, C₁-C₄-Alkylthio, C₁-C₄-Alkylamino, Di-(C₁-C₄-alkyl)amino oder Phenyl,
R²⁰ und R²¹ unabhängig voneinander für
Wasserstoff, C₁-C₄-Alkyl, C₁-C₄-Alkoxy, (C₁-C₄-Alkyl)carbonyl, Phenyl oder Benzoyl und
R²² für
Wasserstoff, C₁-C₄-Alkyl, Allyl oder Phenyl stehen;
Ph steht für
Phenyl, das unsubstituiert sein oder ein bis drei Substituenten tragen kann, ausgewählt aus der Gruppe bestehend aus Halogen, Cyano, Nitro, -S(O)ₙ-R²³, (C₁-C₄-Alkoxy)carbonyl, -SO2-N(R²⁴)R²⁵, -N(R²⁴)-COR²⁵, -N(R²⁴)-SO₂R²⁶, C₁-C₄-Alkyl, C₁-C₄-Alkoxy, C₁-C₄-Halogenalkyl und C₁-C₄-Halogenalkoxy, wobei die vier letztgenannten Reste ihrerseits ein oder zwei der folgenden Substituenten tragen können: C₁-C₄-Alkoxy, C₁-C₄-Alkylthio und/oder Cyano;
m und n stehen unabhängig voneinander für
0, 1 oder 2;
R⁸ und R²³ stehen unabhängig voneinander für
C₁-C₄-Alkyl oder C₁-C₄-Halogenalkyl, die beide ein oder zwei C₁-C₄-Alkoxy, C₁-C₄-Alkylthio- und/oder Cyanoreste tragen können;
R⁹, R¹⁰, R²⁴ und R²⁵ stehen unabhängig voneinander für
Wasserstoff, C₁-C₄-Alkyl, C₁-C₄-Halogenalkyl oder Phenyl, das unsubstituiert sein oder ein bis drei Halogen-, C₁-C₄-Alkyl- und/oder C₁-C₄-Alkoxyreste tragen kann;
R¹¹ und R²⁶ stehen unabhängig voneinander für
C₁-C₄-Alkyl oder C₁-C₄-Halogenalkyl, die beide ein oder zwei Cyano-, Phenyl- und/oder Benzylreste tragen können;
R¹, R², R³ und R⁴
Wasserstoff;
R⁵
Wasserstoff;
R⁶ und R⁷
unabhängig voneinander C₁-C₄-Alkyl, Benzyl oder zusammen eine Ethylen- oder Propylenkette, wobei jede Methyleneinheit ein oder zwei C₁-C₄-Alkylreste tragen kann;
sowie die landwirtschaftlich brauchbaren Salze von I und die Ester von I mit C₁-C₁₀-Carbon-, Sulfon-, Phosphonsäuren oder anorganischen Säuren.

2. Aroylcyclohexandione der Formel I gemäß Anspruch 1, wobei mindestens ein Substituent am Phenyl- oder Heteroarylring Ar einen Rest -N=N-Ph, -S(O)ₘ-R⁸, (C₁-C₄-Alkoxy)carbonyl, -N(R⁹)-SO₂-R¹¹, -SO₂-N(R⁹)R¹⁰, -N(R⁹)-COR¹⁰ oder C₁-C₄-Halogenalkyl bedeutet.

3. Verfahren zur Herstellung von 2-Aroylcyclohexandionen der Formel I gemäß Anspruch 1, dadurch gekennzeichnet, daß man eine Verbindung der Formel II 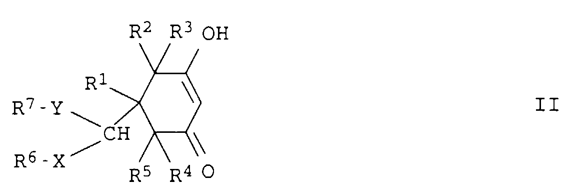 mit einem Säurechlorid der Formel III
L-CO-Ar III
in der L für eine Abgangsgruppe steht,
in Gegenwart einer Base in einem inerten Lösungsmittel umsetzt,
und den hierbei erhaltenen Enolester der Formel IV 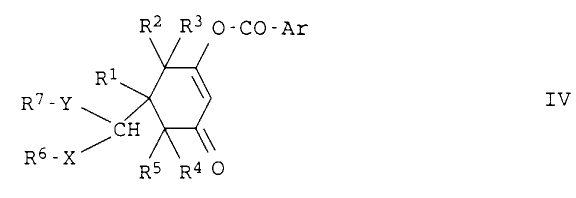 in Gegenwart einer Cyanid-Quelle und einer Base in einem inerten Lösungsmittel umlagert.

4. Verwendung der 2-Aroylcyclohexandione I gemäß Anspruch 1 als Herbizide oder zur Regulierung des Pflanzenwachstums.

5. Herbizides Mittel, enthaltend eine herbizid wirksame Menge mindestens eines 2-Aroylcyclohexandions der Formel I oder ein landwirtschaftlich brauchbares Salz oder einen Ester von I, gemäß Anspruch 1, und mindestens einen inerten flüssigen und/ oder festen Trägerstoff sowie gewünschtenfalls mindestens ein Adjuvants.

6. Mittel zur Regulierung des Pflanzenwachstums, enthaltend eine herbizid wirksame Menge mindestens eines 2-Aroylcyclohexandions der Formel I oder ein landwirtschaftlich brauchbares Salz oder einen Ester von I, gemäß Anspruch 1, und mindestens einen inerten flüssigen und/oder festen Trägerstoff sowie gewünschtenfalls mindestens ein Adjuvants.

7. Verfahren zur Herstellung von herbizid wirksamen Mitteln oder von Mitteln zur Regulierung des Pflanzenwachstums, dadurch gekennzeichnet, daß man eine herbizid wirksame Menge mindestens eines 2-Aroylcyclohexandions der Formel I oder ein landwirtschaftlich brauchbares Salz oder einen Ester von I, gemäß Anspruch 1, und mindestens einen inerten flüssigen und/ oder festen Trägerstoff sowie gewünschtenfalls mindestens ein Adjuvants mischt.

8. Verfahren zur Bekämpfung von unerwünschtem Pflanzenwuchs, dadurch gekennzeichnet, daß man eine herbizid wirksame Menge mindestens eines 2-Aroylcyclohexandions der Formel I, oder eines landwirtschaftlich brauchbaren Salzes oder eines Esters von I, gemäß Anspruch 1, auf Pflanzen, deren Lebensraum oder auf Saatgut einwirken läßt.

9. Verfahren zur Regulierung des Pflanzenwachstums dadurch gekennzeichnet, daß man eine zum Regulieren des Pflanzenwachstums wirksame Menge mindestens eines 2-Aroylcyclohexandions der Formel I, oder eines landwirtschaftlich brauchbaren Salzes oder eines Esters von I, gemäß Anspruch 1, auf Pflanzen, deren Lebensraum oder auf das Saatgut der Pflanzen einwirken läßt.

## Claims

1. A 2-aroylcyclohexanedione of the formula I 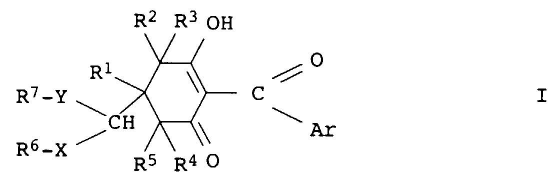 where the variables have the following meanings:
X and Y are oxygen;
Ar is
phenyl or a 5- or 6-membered heteroaryl ring, the phenyl or heteroaryl ring carrying at least one, but at most four substituents, in each case selected from the group consisting of halogen, cyano, nitro, -N=N-Ph, (C₁-C₄-alkoxy)carbonyl, -N(R⁹)-COR¹⁰, -N(R⁹)-SO₂-R¹¹, -SO₂-N(R⁹)R¹⁰, -S(O)ₘ-R⁸, C₁-C₄-alkyl, C₁-C₄-alkoxy, C₁-C₄-haloalkyl and C₁-C₄-haloalkoxy, it being possible for the four last-mentioned radicals in turn to carry one or two of the following substituents: C₁-C₄-alkoxy, C₁-C₄-alkylthio and/or cyano,
and it being possible for two adjacent C atoms of the phenyl or heteroaryl ring also to be bridged by means of a chain -C(R¹²)=C(R¹³)-C(R¹⁴)=C(R¹⁵)-, -Z¹-C(R¹²)=N-, -Z¹-N=C(R¹²)-, -Z¹-C(R¹²)=C(R¹³)-, -Z¹-C(R¹²)=C(R¹³)-C(R¹⁴, R¹⁵)-, -Z¹-C(R¹², R¹³)-C(R¹⁴, R¹⁵)-, -Z¹-C(R¹², R¹³)-C(R¹⁴, R¹⁵)-Z²-, -C(R¹², R¹³)-Z¹-C(R¹⁴, R¹⁵)-C(R¹⁶, R¹⁷)-, -Z¹-N(R²⁰)-Z²-, -Z¹-Z²-N(R²⁰)-, -Z¹-C(R¹², R¹³)-Z²-N(R²⁰)-, -Z¹-N(R²⁰)-Z²-N(R²¹)-, -N(R²¹)-Z¹-Z²-, -N(R²⁰)-Z¹-N=C(R¹²)-, -Z¹-C(R¹², R¹³)-C(=NOR²²)-, -Z¹-C(R¹², R¹³)-C(R¹⁴, R¹⁵)-C(=NOR²²)- or Z¹-Z²-Z¹, where
Z¹ and Z² independently of one another are
oxygen, sulfur, -SO-, -SO₂-, -CO-, -C(R¹⁸, R¹⁹)- or -N(R²⁰)- and where
R¹² to R¹⁹ independently of one another are
hydrogen, halogen, hydroxyl, C₁-C₄-alkyl, C₁-C₄-alkoxy, C₁-C₄-alkylthio, C₁-C₄-alkylamino, di-(C₁-C₄-alkyl)amino or phenyl,
R²⁰ and R²¹ independently of one another are
hydrogen, C₁-C₄-alkyl, C₁-C₄-alkoxy, (C₁-C₄-alkyl)carbonyl, phenyl or benzoyl and
R²² is
hydrogen, C₁-C₄-alkyl, allyl or phenyl;
Ph is
phenyl which can be unsubstituted or can carry one to three substituents selected from the group consisting of halogen, cyano, nitro, -S(O)ₙ-R²³, (C₁-C₄-alkoxy)carbonyl, -SO₂-N(R²⁴)R²⁵, -N(R²⁴)-COR²⁵, -N(R²⁴)-SO₂R²⁶, C₁-C₄-alkyl, C₁-C₄-alkoxy, C₁-C₄-haloalkyl and C₁-C₄-haloalkoxy, it being possible for the four last-mentioned radicals in turn to carry one or two of the following substituents: C₁-C₄-alkoxy, C₁-C₄-alkylthio and/or cyano;
m and n independently of one another are
0, 1 or 2;
R⁸ and R²³ independently of one another are
C₁-C₄-alkyl or C₁-C₄-haloalkyl, which both can carry one or two C₁-C₄-alkoxy, C₁-C₄-alkylthio and/or cyano radicals;
R⁹, R¹⁰, R²⁴ and R²⁵ independently of one another are
hydrogen, C₁-C₄-alkyl, C₁-C₄-haloalkyl or phenyl, which can be unsubstituted or can carry one to three halogen, C₁-C₄-alkyl and/or C₁-C₄-alkoxy radicals;
R¹¹ and R²⁶ independently of one another are
C₁-C₄-alkyl or C₁-C₄-haloalkyl, which both can carry one or two cyano, phenyl and/or benzyl radicals;
R¹, R², R³ and R⁴
are hydrogen;
R⁵ is
hydrogen;
R⁶ and R⁷
independently of one another are C₁-C₄-alkyl, benzyl or together are an ethylene or propylene chain, it being possible for each methylene unit to carry one or two C₁-C₄-alkyl radicals;
or the agriculturally utilizable salts of I and the esters of I with C₁-C₁₀-carboxylic, sulfonic or phosphonic acids or inorganic acids.

2. A 2-aroylcyclohexanedione of the formula I as claimed in claim 1, at least one substituent on the phenyl or heteroaryl ring Ar denoting a radical -N=N-Ph, -S(O)ₘ-R⁸, (C₁-C₄-alkoxy)-carbonyl, -N(R⁹)-SO₂-R¹¹, -SO₂-N(R⁹)R¹⁰, -N(R⁹)-COR¹⁰ or C₁-C₄-haloalkyl.

3. A process for preparing 2-aroylcyclohexanediones of the formula I as claimed in claim 1, which comprises reacting a compound of the formula II 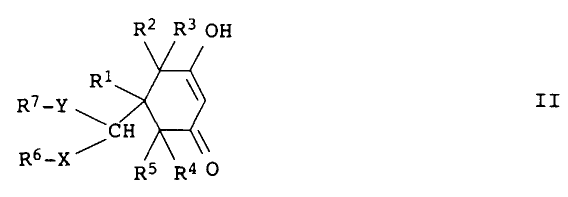 with an acid chloride of the formula III
L-CO-Ar III
where L is a leaving group,
in an inert solvent in the presence of a base,
and rearranging the enol ester, obtained in this process, of the formula IV 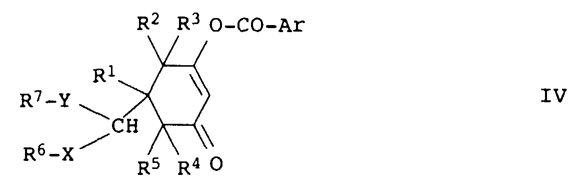 in an inert solvent in the presence of a cyanide source and of a base.

4. The use of the 2-aroylcyclohexanediones I as claimed in claim 1 as herbicides or for regulating plant growth.

5. A herbicidal composition, containing a herbicidally active amount of at least one 2-aroylcyclohexanedione of the formula I or an agriculturally utilizable salt or an ester of I, as claimed in claim 1, and at least one inert liquid and/or solid carrier and, if desired, at least one adjuvant.

6. A composition for regulating plant growth, containing a herbicidally active amount of at least one 2-aroylcyclohexanedione of the formula I or an agriculturally utilizable salt or an ester of I, as claimed in claim 1, and at least one inert liquid and/or solid carrier and, if desired, at least one adjuvant.

7. A process for preparing herbicidally active compositions or compositions for regulating plant growth, which comprises mixing a herbicidally active amount of at least one 2-aroylcyclohexanedione of the formula I or an agriculturally utilizable salt or an ester of I, as claimed in claim 1, and at least one inert liquid and/or solid carrier and, if desired, at least one adjuvant.

8. A method of controlling undesired plant growth, which comprises allowing a herbicidally active amount of at least one 2-aroylcyclohexanedione of the formula I, or of an agriculturally utilizable salt or of an ester of I, as claimed in claim 1, to act on plants, their habitat or on seed.

9. A method of regulating plant growth, which comprises allowing an amount of at least one 2-aroylcyclohexanedione of the formula I effective for regulating plant growth, or of an agriculturally utilizable salt or of an ester of I, as claimed in claim 1, to act on plants, their habitat or on the seed.

## Revendications

1. 2-aroylcyclohexanediones de formule I 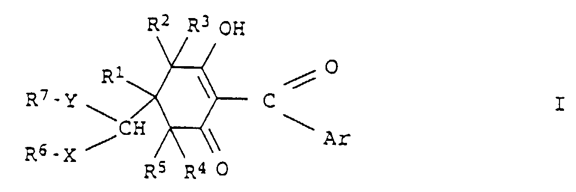 dans laquelle les symboles ont les significations suivantes :
X et Y : l'oxygène ;
Ar
un groupe phényle ou un cycle hétéroaryle à cinq ou six chaînons, chacun de ces cycles portant au moins un et au maximum quatre substituants choisis parmi les halogènes, les groupes cyano, nitro, -N=N-Ph, (alcoxy en C1-C4)carbonyle, -N(R⁹)-COR¹⁰, -N(R⁹)-SO₂-R¹¹, -SO₂-N(R⁹)R¹⁰, -S(O)ₘ-R⁸, alkyle en C1-C4, alcoxy en C1-C4, halogénoalkyle en C1-C4 et halogénoalcoxy en C1-C4, ces quatre derniers substituants pouvant eux-mêmes porter un ou deux des substituants suivants : alcoxy en C1-C4, alkylthio en C1-C4 et/ou cyano, cependant que deux atomes de carbone voisins du cycle phényle ou hétéroaryle peuvent être reliés entre eux par un pont consistant en une chaîne -C(R¹²)=C(R¹³)-C(R¹⁴)=C(R¹⁵)-, -Z¹-C(R¹²)=N-, -Z¹-N=C(R¹²)-, -Z¹-C(R¹²)=C(R¹³)-, -Z¹-C(R¹²)=C(R¹³)-C(R¹⁴, R¹⁵)-, -Z¹-C(R¹², R¹³)-C(R¹⁴, R¹⁵)-, -Z¹-C(R¹², R¹³)-C(R¹⁴, R¹⁵)-Z²-, -C(R¹², R¹³)-Z¹-C(R¹⁴, R¹⁵)-C(R¹⁶, R¹⁷)-, -Z¹-N(R²⁰)-Z²-, -Z¹-Z²-N(R²⁰)-, -Z¹-C(R¹², R¹³)-Z²-N(R²⁰)-, -Z¹-N(R²⁰)-Z²-N(R²¹)-, -N(R²¹)-Z¹-Z²-, -N(R²⁰)-Z¹-N=C(R¹²)-, -Z¹-C(R¹², R¹³)-C(=NOR²²)-, -Z¹-C(R¹², R¹³)-C(R¹⁴, R¹⁵)-C(=NOR²²)- ou Z¹-Z²-Z¹, dans laquelle
Z¹ et Z² représentent chacun, indépendamment l'un de l'autre l'oxygène, le soufre, -SO-, -SO₂-, -CO-, -C(R¹⁸, R¹⁹)- ou -N(R²⁰)-,
R¹² à R¹⁹ représentent chacun, indépendamment les uns des autres, l'hydrogène, un halogène, un groupe hydroxy, alkyle en C1-C4, alcoxy en C1-C4, alkylkylthio en C1-C4, alkylamino en C1-C4, di-(alkyle en C1-C4)amino ou phényle,
R²⁰ et R²¹ représentent chacun, indépendamment l'un de l'autre, l'hydrogène, un groupe alkyle en C1-C4, alcoxy en C1-C4, (alkyle en C1-C4)carbonyle, phényle ou benzoyle et
R²² représente
l'hydrogène, un groupe alkyle en C1-C4, allyle ou phényle ;
Ph représente
un groupe phényle non substitué ou portant un à trois substituants choisis parmi les halogènes, les groupes cyano, nitro, -S(O)n-R²³, (alcoxy en C1-C4)carbonyle, -SO₂-N(R²⁴)R²⁵, -N(R²⁴)-COR²⁵, -N(R²⁴)-SO₂R²⁶, alkyle en C1-C4, alcoxy en C1-C4, halogénoalkyle en C1-C4 ou halogénoalcoxy en C1-C4, ces quatre derniers groupes pouvant eux-mêmes porter un ou deux des substituants suivants : alcoxy en C1-C4, alkylthio en C1-C4 et/ou cyano ;
m et n sont des nombres égaux chacun, indépendamment l'un de l'autre à 0, 1 ou 2 ;
R⁸ et R²³ représentent chacun, indépendamment l'un de l'autre, un groupe alkyle en C1-C4 ou halogénoalkyle en C1-C4, chacun d'eux pouvant porter un ou deux substituants alcoxy en C1-C4, alkylthio en C1-C4 et/ou cyano ;
R⁹, R¹⁰, R²⁴ et R²⁵ représentent chacun, indépendamment les uns des autres, l'hydrogène, un groupe alkyle en C1-C4, halogénoalkyle en C1-C4 ou phényle, lequel peut être substitué ou peut porter un à trois substituants halogéno, alkyle en C1-C4 et/ou alcoxy en C1-C4 ;
R¹¹ et R²⁶ représentent chacun, indépendamment l'un de l'autre un groupe alkyle en C1-C4 ou halogénoalkyle en C1-C4, chacun d'eux pouvant porter un ou deux substituants cyano, phényle et/ou benzyle ;
R¹, R², R³ et R⁴ représentent
l'hydrogène ;
R⁵ représente
l'hydrogène ;
R⁶ et R⁷ représentent chacun, indépendamment l'un de l'autre, un groupe alkyle en C1-C4, benzyle, ou bien, ensemble, une chaîne éthylène ou propylène dont chaque motif méthylène peut porter un ou deux substituants alkyle en C1-C4 ;
et les sels de I et les esters de I et d'acides carboxyliques en C1-C10, d'acides sulfoniques, d'acides phosphoniques ou d'acides minéraux, convenant pour les applications agricoles.

2. 2-aroylcyclohexanediones de formule I selon la revendication 1, pour lesquelles au moins un substituant du cycle phényle ou hétéroaryle Ar consiste en un groupe -N=N-Ph, -S(O)ₘ-R⁸, (alcoxy en C1-C4)carbonyle, -N(R⁹)-SO₂-R¹¹, -SO₂-N(R⁹)R¹⁰, -N(R⁹)-COR¹⁰ ou halogénoalkyle en C1-C4.

3. Procédé de préparation des 2-aroylcyclohexanediones de formule I de la revendication 1, caractérisé par le fait que l'on fait réagir un composé de formule II 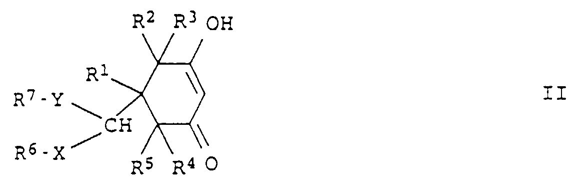 avec un chlorure d'acide de formule III
L-CO-Ar III
dans laquelle L représente un groupe éliminable,
en présence d'une base, dans un solvant inerte,
ce qui donne un ester d'énol de formule IV 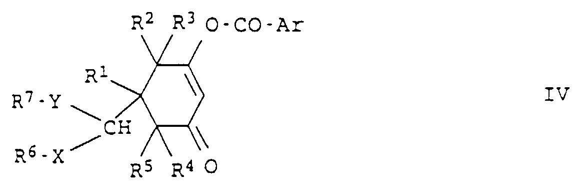 qu'on soumet à transposition en présence d'une source de cyanure et d'une base dans un solvant inerte.

4. Utilisation des 2-aroylcyclohexanediones I de la revendication 1 en tant qu'herbicides ou pour la régulation de la croissance des végétaux.

5. Produit herbicide contenant une quantité herbicide efficace d'au moins une 2-aroylcyclohexanedione de formule I ou d'un sel ou d'un ester de I, selon la revendication 1, convenant pour les applications agricoles, et au moins un véhicule liquide et/ou solide inerte et le cas échéant au moins un adjuvant.

6. Produit pour la régulation de la croissance des végétaux, contenant une quantité herbicide efficace d'au moins une 2-aroylcyclohexanedione de formule I ou d'un sel ou ester de I apte aux applications agricoles, selon la revendication 1, et au moins un véhicule liquide et/ou solide inerte et le cas échéant au moins un adjuvant.

7. Procédé pour préparer des produits herbicides ou des produits régulateurs de la croissance des végétaux, caractérisé par le fait que l'on mélange une quantité herbicide efficace d'au moins une 2-aroylcyclohexanedione de formule I ou d'un sel ou ester de I apte aux applications agricoles, selon la revendication 1, et au moins un véhicule liquide et/ou solide inerte et le cas échéant au moins un adjuvant.

8. Procédé pour combattre les croissances de végétaux indésirables, caractérisé par le fait que l'on fait agir une quantité efficace d'au moins une 2-aroylcyclohexanedione de formule I ou d'un sel ou ester de I apte aux applications agricoles, selon la revendication 1, sur les végétaux, leur habitat ou sur les semences.

9. Procédé pour la régulation de la croissance des végétaux, caractérisé par le fait que l'on fait agir une quantité régulatrice efficace de la croissance des végétaux d'au moins une 2-aroylcyclohexanedione de formule I ou d'un sel ou ester de I apte aux applications agricoles, selon la revendication 1, sur les végétaux, leur habitat ou sur les semences des végétaux.
